# EUROPEAN PATENT APPLICATION

(11) **EP 4 714 988 A1**
(43) Date of publication of application: **25.03.2026**
(21) Application number: 24815615.0
(22) Date of filing: 31.05.2024
(51) Int. Cl.: C08F 20/22, C07C 31/36, C07C 31/42, C07C 67/08, C07C 69/653, C08F 220/22, C08F 220/38, C08L 33/16, G03F 7/004, G03F 7/038, G03F 7/039, G03F 7/20

(54) **FLUORINE-CONTAINING POLYMER, COMPOSITION FOR FORMING FLUORINE-CONTAINING RESIN FILM, FLUORINE-CONTAINING RESIN FILM, COMPOSITION FOR FORMING RESIST PATTERN, METHOD FOR FORMING RESIST PATTERN, COMPOSITION FOR FORMING RESIST UPPER LAYER FILM, METHOD FOR DECOMPOSING FLUORINE-CONTAINING POLYMER, FLUORINE-CONTAINING POLYMERIZABLE MONOMER, COMPOUND, AND METHOD FOR PRODUCING FLUORINE-CONTAINING POLYMERIZABLE MONOMER**

(30) Priority: 31.05.2023 JP 2023090233; 15.12.2023 JP 2023212231
(71) Applicant: Central Glass Company, Limited, Ube-shi, Yamaguchi 755-0001 (JP)
(72) Inventor: MASUBUCHI, Takashi, Tokyo 101-0054 (JP); KANEKO, Yuzuru, Tokyo 101-0054 (JP); IMAIZUMI, Yuki, Tokyo 101-0054 (JP)
(74) Representative: Manitz Finsterwald Patent- und Rechtsanwaltspartnerschaft mbB
(86) International application number: PCT/JP2024/020043
(87) International publication number: WO 2024/248135

(57) **Abstract**

Provided is a fluorine-containing polymer that has water repellency and photosensitivity equivalent to those of conventional fluorine-containing polymers but can be decomposed at a lower temperature. A fluorine-containing polymer of the present disclosure contains a repeating unit represented by the following formula (1) or (1)', wherein, in formula (1), R¹ is a hydrogen atom, a fluorine atom, a chlorine atom, or a C1-C10 linear or C3-C10 branched alkyl group, some or all of the hydrogen atoms bonded to the carbon atoms in the alkyl group being optionally replaced by fluorine atoms; in formula (1), R² is a single bond, an alkylene group optionally having a linear, branched, or cyclic structure, an aromatic ring, an ester, a carbonyl, an ether, an amide, an amine, or a composite substituent thereof, a part of which is optionally fluorinated and/or chlorinated; in formula (1)', R^{1a} is a C1-C10 linear or C3-C10 branched alkyl group, some or all of the hydrogen atoms bonded to the carbon atoms in the alkyl group being optionally replaced by fluorine atoms and/or chlorine atoms; in formula (1)', R^{2a} is an alkylene group optionally having a linear, branched, or cyclic structure, an aromatic ring, an ester, a carbonyl, an ether, an amide, an amine, or a composite substituent thereof, a part of which is optionally fluorinated and/or chlorinated; in formula (1)', R^{2b} is an alkylene group optionally having a linear, branched, or cyclic structure, an aromatic ring, an ester, a carbonyl, an ether, an amide, an amine, or a composite substituent thereof, a part of which is optionally fluorinated and/or chlorinated; in formulae (1) and (1)', X is a hydroxy group, an alkoxy group, an oxycarbonyl group, an oxysulfonyl group, an oxycarboxy group, or a hydrogen atom; and in formulae (1) and (1)', n is a natural number of 1 to 3.

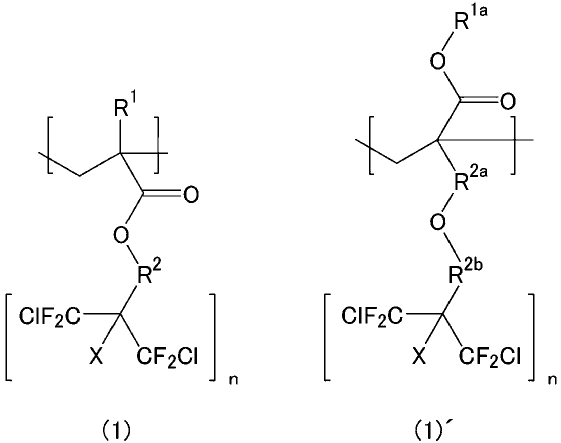

## Description

### TECHNICAL FIELD

The present disclosure relates to a fluorine-containing polymer, a composition for forming a fluorine-containing resin film, a fluorine-containing resin film, a composition for forming a resist pattern, a method for forming a resist pattern, a composition for forming a resist upper layer film, a method for decomposing a fluorine-containing polymer, a fluorine-containing polymerizable monomer, a compound, and a method for producing a fluorine-containing polymerizable monomer.

### BACKGROUND ART

Fluorine-containing polymers (fluorine-containing compounds) have continued to be used and developed across a wide range of application fields, primarily in the field of advanced materials, due to the characteristics of fluorine, such as water repellency, oil repellency, low water absorption, heat resistance, weather resistance, corrosion resistance, transparency, photosensitivity, low refractive index, and low dielectric constant. In particular, regarding coating applications, active research and development have been conducted in fields such as antireflection films that utilize low refractive index and visible light transparency, optical devices that utilize transparency in high-wavelength bands (optical communication wavelength bands), and resist materials that utilize transparency in the ultraviolet region (particularly in the vacuum ultraviolet wavelength region). A common polymer design in these application fields is to introduce as much fluorine as possible to achieve transparency at operating wavelengths, while also achieving adhesion to substrates and a high glass transition point (hardness).

As a monomer for synthesizing such a fluorine-containing polymer, Patent Literature 1 describes a polymerizable monomer represented by the following formula (12): 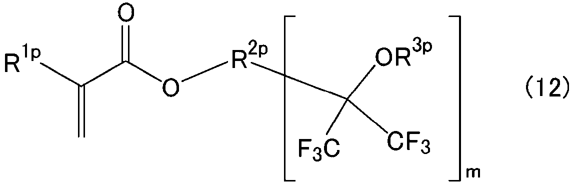 wherein, in formula (12), R^{1p} represents a group selected from the group consisting of a hydrogen atom, a halogen atom, a hydrocarbon group, and a fluorine-containing alkyl group (the fluorine-containing alkyl group is linear or branched and may contain a cyclic structure); R^{2p} is a divalent or trivalent organic group which is selected from an aliphatic hydrocarbon group (the aliphatic hydrocarbon group is linear or branched and may contain a cyclic structure), an aromatic ring group, or a composite substituent thereof, some or all of the hydrogen atoms of the group being optionally replaced by fluorine atoms or hydroxy groups; R^{3p} is a hydrogen atom, a hydrocarbon group, a fluorine-containing alkyl group (the fluorine-containing alkyl group is linear or branched and may contain a cyclic structure), or an aromatic ring group, and the hydrocarbon group or the fluorine-containing alkyl group is optionally intervened by a divalent linking group selected from an ether group (-O-) or a carbonyl group (-(C=O)-); and m represents an integer of 1 to 2, and when m is 2, the two R^{3p} may be identical to each other or may be different from each other.

The polymerizable monomer represented by formula (12) has a (CF₃)₂(OR^{3p})C-moiety derived from hexafluoroacetone, and is a monomer compound with a high fluorine content, into which polar groups are successfully introduced in a well-balanced manner within the molecule.

This polymerizable monomer also exhibits excellent polymerizability, and a fluorine-containing polymer obtained by polymerizing this polymerizable monomer combines the transparency imparted by fluorine atoms with the adhesion and processability imparted by polar groups, and is known to have excellent physical properties as an antireflection film material, an optical device material, a resist material, or the like.

Meanwhile, since resist materials become waste material after the photolithography step, material design that considers the disposal method of such materials is also required. Patent Literature 2 teaches that a polymer containing a repeating unit formed from a monomer represented by the following formula (13) has the characteristic that "since the (meth)acrylic acid ester moiety can be alkali-hydrolyzed during resist waste liquid treatment following device fabrication, the polymer may be converted into a lower molecular weight compound with low accumulation potential, and it also exhibits high combustibility upon combustion disposal due to its low fluorine substitution ratio".

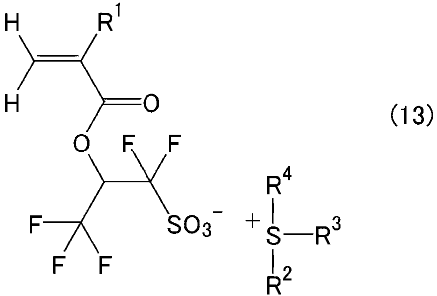

### CITATION LIST

### - Patent Literature

Patent Literature 1: JP 4083399 B
Patent Literature 2: JP 2012-107151 A

### SUMMARY OF INVENTION

### - Technical Problem

Photosensitive compositions containing a fluorine-containing polymer as the main component are widely used to form a resist film and/or an upper layer film for protecting the resist film in a photolithography step. The resist film and the upper layer film are removed from the substrate in an alkali development step or a resist removal step. The material (waste material) that was the resist film or the upper layer film is decomposed so as not to affect the environment. The fluorine-containing polymer is the main component of the waste material, and the evaluation of the decomposition of the waste material substantially corresponds to the evaluation of the decomposition of the fluorine-containing resin. The decomposition of the waste material is preferably thermal decomposition because it is simple. Then, the amount of energy applied for the thermal decomposition is preferably small.

According to the teachings of Patent Literature 2, it is considered that lowering the fluorine substitution ratio is a point for thermal decomposition of the compound. On the other hand, a plurality of fluorine atoms impart polarity to the compound, making it useful as a resist material or the like. In Patent Literature 2, it is considered that a balance between combustibility and usefulness as a resist material or the like is achieved by localizing fluorine to a specific group. Following this teaching leads to the idea that the compound disclosed in Patent Literature 1 achieves a balance between combustibility and usefulness as a resist material or the like.

Accordingly, a breakthrough is necessary in the design of a compound that enables a reduction in the amount of energy required for the thermal decomposition of a fluorine-containing polymer that has become waste material after the photolithography step.

An object of the present disclosure is to provide a fluorine-containing polymer that has water repellency and photosensitivity equivalent to those of conventional fluorine-containing polymers but can be decomposed at a lower temperature.

### - Solution to Problem

As shown in the examples of the present specification, the present inventors have newly synthesized a fluorine-containing polymer having a skeleton in which a chlorodifluoro group is bonded at a specific position.

Embodiment (1) of the present disclosure is a fluorine-containing polymer, containing a repeating unit represented by the following formula (1) or (1)': 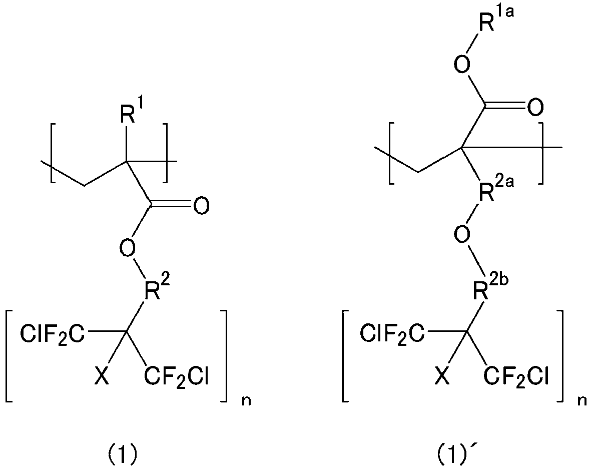
wherein, in formula (1), R¹ is a hydrogen atom, a fluorine atom, a chlorine atom, or a C1-C10 linear or C3-C10 branched alkyl group, some or all of hydrogen atoms bonded to carbon atoms in the alkyl group being optionally replaced by fluorine atoms;
in formula (1), R² is a single bond, an alkylene group optionally having a linear, branched, or cyclic structure, an aromatic ring, an ester, a carbonyl, an ether, an amide, an amine, or a composite substituent thereof, a part of which is optionally fluorinated, chlorinated, or both fluorinated and chlorinated;
in formula (1)', R^{1a} is a C1-C10 linear or C3-C10 branched alkyl group, some or all of hydrogen atoms bonded to carbon atoms in the alkyl group being optionally replaced by fluorine atoms, chlorine atoms, or both;
in formula (1)', R^{2a} is an alkylene group optionally having a linear, branched, or cyclic structure, an aromatic ring, an ester, a carbonyl, an ether, an amide, an amine, or a composite substituent thereof, a part of which is optionally fluorinated, chlorinated, or both fluorinated and chlorinated;
in formula (1)', R^{2b} is an alkylene group optionally having a linear, branched, or cyclic structure, an aromatic ring, an ester, a carbonyl, an ether, an amide, an amine, or a composite substituent thereof, a part of which is optionally fluorinated, chlorinated, or both fluorinated and chlorinated;
in formulae (1) and (1)', X is a hydroxy group, an alkoxy group, an oxycarbonyl group, an oxysulfonyl group, an oxycarboxy group, or a hydrogen atom; and in formulae (1) and (1)', n is a natural number of 1 to 3.

Embodiment (2) of the present disclosure is the fluorine-containing polymer according to Embodiment (1), wherein the repeating units represented by formulae (1) and (1)' include repeating units represented by the following formulae (2) and (2)', respectively.

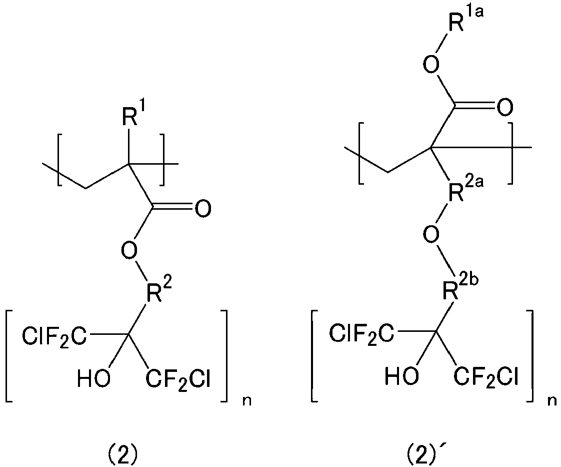

Embodiment (3) of the present disclosure is the fluorine-containing polymer according to Embodiment (2), containing a repeating unit represented by the following formula (3): 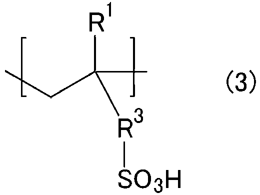 wherein, in formula (3), R³ is a single bond, an alkylene group optionally having a linear, branched, or cyclic structure, or an aromatic ring, a part of which optionally contains an ester bond, a carbonyl bond, an ether bond, an amide bond, an acetal bond, a hydroxy group, an amino group, a fluorine atom, or a chlorine atom, and is optionally a composite substituent thereof.

Embodiment (4) of the present disclosure is the fluorine-containing polymer according to any one of Embodiments (1) to (3), wherein the repeating units represented by formulae (1) and (1)' include repeating units represented by the following formulae (4) and (4)', respectively.

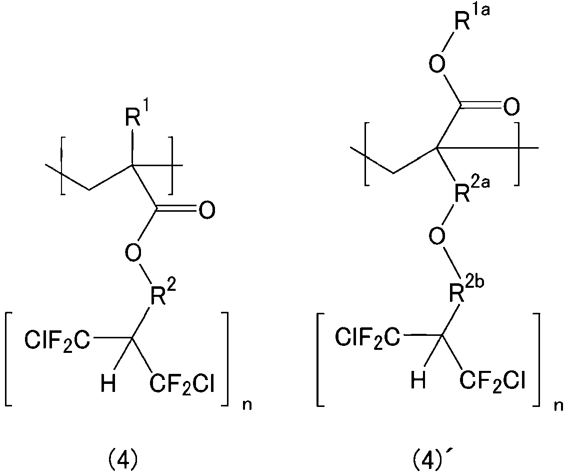

Embodiment (5) of the present disclosure is the fluorine-containing polymer according to Embodiment (4), containing a repeating unit represented by the following formula (5): 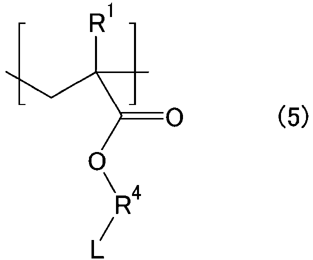
wherein, in formula (5), R⁴ is a single bond, an alkylene group optionally having a linear, branched, or cyclic structure, or an aromatic ring, a part of which optionally contains an ester bond, a carbonyl bond, an ether bond, an amide bond, an acetal bond, a hydroxy group, an amino group, a fluorine atom, or a chlorine atom, and is optionally a composite substituent thereof; and
L is an acid-dissociable group.

Embodiment (6) of the present disclosure is the fluorine-containing polymer according to any one of Embodiments (1) to (4), wherein the repeating units represented by formulae (1) and (1)' include repeating units represented by the following formulae (2) and (2)', respectively.

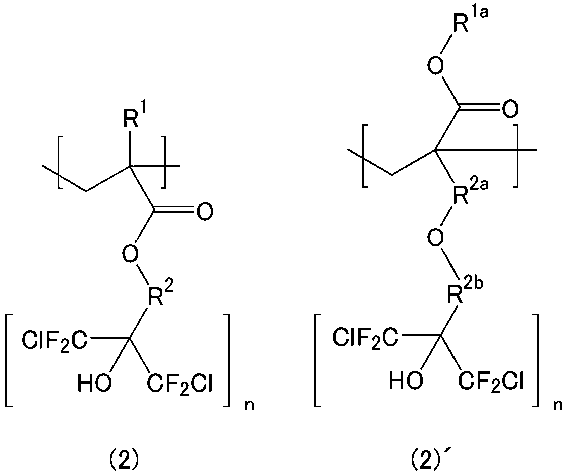

Embodiment (7) of the present disclosure is the fluorine-containing polymer according to Embodiment (6), containing a repeating unit represented by the following formula (5): 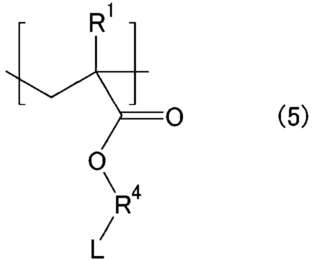
wherein, in formula (5), R⁴ is a single bond, an alkylene group optionally having a linear, branched, or cyclic structure, or an aromatic ring, a part of which optionally contains an ester bond, a carbonyl bond, an ether bond, an amide bond, an acetal bond, a hydroxy group, an amino group, a fluorine atom, or a chlorine atom, and is optionally a composite substituent thereof; and
L is an acid-dissociable group.

Embodiment (8) of the present disclosure is a composition for forming a fluorine-containing resin film, containing the fluorine-containing polymer according to any one of Embodiments (1) to (7).

Embodiment (9) of the present disclosure is a fluorine-containing resin film, including a coating film of the composition for forming a fluorine-containing resin film according to Embodiment (8).

In the present specification, the term "coating film" includes a cured product in which a fluorine-containing polymer has been cured by a polymerization initiator, and a film-like product obtained by drying a composition for forming a fluorine-containing resin film containing a fluorine-containing polymer and a solvent.

Embodiment (10) of the present disclosure is a composition for forming a resist pattern, containing the fluorine-containing polymer according to any one of Embodiments (1) to (7), an acid generator, and a solvent.

Embodiment (11) of the present disclosure is a method for forming a resist pattern, the method including: a step of providing the composition for forming a resist pattern according to Embodiment (10); a film formation step including applying the composition for forming a resist pattern onto a substrate to form a film; an exposure step including exposing the film to electromagnetic waves or high-energy rays having a wavelength of 300 nm or less through a photomask to transfer a pattern of the photomask to the film; and a development step including developing the film using a developer to obtain the pattern.

Embodiment (12) of the present disclosure is a composition for forming a resist upper layer film, containing the fluorine-containing polymer according to any one of Embodiments (1) to (7) and a solvent.

Embodiment (13) of the present disclosure is a method for forming a resist pattern, the method including: a step of providing the composition for forming a resist upper layer film according to Embodiment (12); a film formation step including applying the composition for forming a resist upper layer film onto a resist film to form a resist upper layer film; an exposure step including exposing the resist upper layer film and the resist film to electromagnetic waves or high-energy rays having a wavelength of 300 nm or less through a photomask to transfer a pattern of the photomask to the resist film; and a development step including removing the resist upper layer film and developing the resist film using a developer to obtain the pattern.

Embodiment (14) of the present disclosure is a method for decomposing the fluorine-containing polymer according to any one of Embodiments (1) to (7), the method including heating the fluorine-containing polymer at a temperature in a range of 50°C to 200°C.

Embodiment (15) of the present disclosure is a fluorine-containing polymerizable monomer, represented by the following formula (6) or (6)': 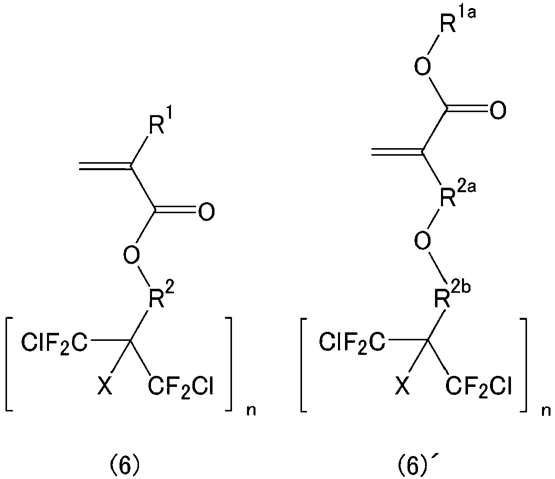
wherein, in formula (6), R¹ is a hydrogen atom, a fluorine atom, a chlorine atom, or a C1-C10 linear or C3-C10 branched alkyl group, some or all of hydrogen atoms bonded to carbon atoms in the alkyl group being optionally replaced by fluorine atoms;
in formula (6), R² is a single bond, an alkylene group optionally having a linear, branched, or cyclic structure, an aromatic ring, an ester, a carbonyl, an ether, an amide, an amine, or a composite substituent thereof, a part of which is optionally fluorinated, chlorinated, or both fluorinated and chlorinated;
in formula (6)', R^{1a} is a C1-C10 linear or C3-C10 branched alkyl group, some or all of hydrogen atoms bonded to carbon atoms in the alkyl group being optionally replaced by fluorine atoms, chlorine atoms, or both;
in formula (6)', R^{2a} is an alkylene group optionally having a linear, branched, or cyclic structure, an aromatic ring, an ester, a carbonyl, an ether, an amide, an amine, or a composite substituent thereof, a part of which is optionally fluorinated, chlorinated, or both fluorinated and chlorinated;
in formula (6)', R^{2b} is an alkylene group optionally having a linear, branched, or cyclic structure, an aromatic ring, an ester, a carbonyl, an ether, an amide, an amine, or a composite substituent thereof, a part of which is optionally fluorinated, chlorinated, or both fluorinated and chlorinated;
in formulae (6) and (6)', X is a hydroxy group, an alkoxy group, an oxycarbonyl group, an oxysulfonyl group, an oxycarboxy group, or a hydrogen atom; and in formulae (6) and (6)', n is a natural number of 1 to 3.

Embodiment (16) of the present disclosure is a compound, represented by the following formula (7): 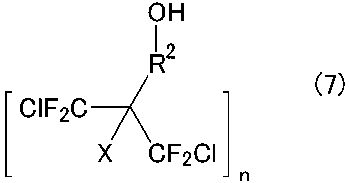
wherein, in formula (7), R² is a single bond, an alkylene group optionally having a linear, branched, or cyclic structure, an aromatic ring, an ester, a carbonyl, an ether, an amide, an amine, or a composite substituent thereof, a part of which is optionally fluorinated, chlorinated, or both fluorinated and chlorinated;
X is a hydroxy group, an alkoxy group, an oxycarbonyl group, an oxysulfonyl group, an oxycarboxy group, or a hydrogen atom; and
n is a natural number of 1 to 3.

Embodiment (17) of the present disclosure is a method for producing a fluorine-containing polymerizable monomer represented by the following formula (6), the method including: a step of providing a compound represented by the following formula (7); and a step of reacting the compound represented by formula (7) with at least one selected from the group consisting of an acid halide, an acid anhydride, an ester, and a carboxylic acid: 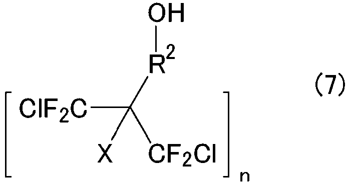
wherein, in formula (7), R² is a single bond, an alkylene group optionally having a linear, branched, or cyclic structure, an aromatic ring, an ester, a carbonyl, an ether, an amide, an amine, or a composite substituent thereof, a part of which is optionally fluorinated, chlorinated, or both fluorinated and chlorinated;
X is a hydroxy group, an alkoxy group, an oxycarbonyl group, an oxysulfonyl group, an oxycarboxy group, or a hydrogen atom; and
n is a natural number of 1 to 3, 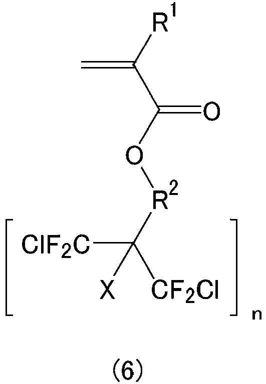
wherein, in formula (6), R¹ is a hydrogen atom, a fluorine atom, a chlorine atom, or a C1-C10 linear or C3-C10 branched alkyl group, some or all of hydrogen atoms bonded to carbon atoms in the alkyl group being optionally replaced by fluorine atoms;
R² is a single bond, an alkylene group optionally having a linear, branched, or cyclic structure, an aromatic ring, an ester, a carbonyl, an ether, an amide, an amine, or a composite substituent thereof, a part of which is optionally fluorinated, chlorinated, or both fluorinated and chlorinated;
X is a hydroxy group, an alkoxy group, an oxycarbonyl group, an oxysulfonyl group, an oxycarboxy group, or a hydrogen atom; and
n is a natural number of 1 to 3.

Embodiment (18) of the present disclosure is a method for producing a fluorine-containing polymerizable monomer represented by the following formula (6)', the method including: a step of providing a compound represented by the following formula (8); and a step of reacting the compound represented by formula (8) with a compound represented by the following formula (9): 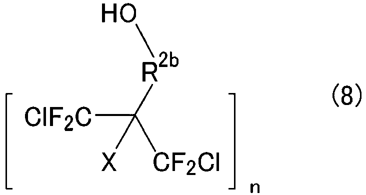
wherein, in formula (8), R^{2b} is an alkylene group optionally having a linear, branched, or cyclic structure, an aromatic ring, an ester, a carbonyl, an ether, an amide, an amine, or a composite substituent thereof, a part of which is optionally fluorinated, chlorinated, or both fluorinated and chlorinated;
in formula (8), X is a hydroxy group, an alkoxy group, an oxycarbonyl group, an oxysulfonyl group, an oxycarboxy group, or a hydrogen atom; and in formula (8), n is a natural number of 1 to 3, 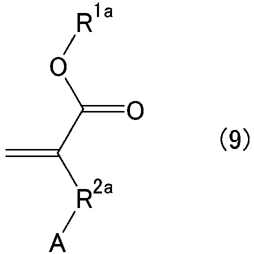
wherein, in formula (9), R^{1a} is a C1-C10 linear or C3-C10 branched alkyl group, some or all of hydrogen atoms bonded to carbon atoms in the alkyl group being optionally replaced by fluorine atoms, chlorine atoms, or both;
in formula (9), R^{2a} is an alkylene group optionally having a linear, branched, or cyclic structure, an aromatic ring, an ester, a carbonyl, an ether, an amide, an amine, or a composite substituent thereof, a part of which is optionally fluorinated, chlorinated, or both fluorinated and chlorinated; and
in formula (9), A is a leaving group, 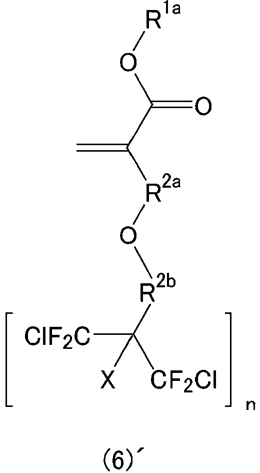
wherein, in formula (6)', R^{1a} is a C1-C10 linear or C3-C10 branched alkyl group, some or all of hydrogen atoms bonded to carbon atoms in the alkyl group being optionally replaced by fluorine atoms, chlorine atoms, or both;
in formula (6)', R^{2a} is an alkylene group optionally having a linear, branched, or cyclic structure, an aromatic ring, an ester, a carbonyl, an ether, an amide, an amine, or a composite substituent thereof, a part of which is optionally fluorinated, chlorinated, or both fluorinated and chlorinated;
in formula (6)', R^{2b} is an alkylene group optionally having a linear, branched, or cyclic structure, an aromatic ring, an ester, a carbonyl, an ether, an amide, an amine, or a composite substituent thereof, a part of which is optionally fluorinated, chlorinated, or both fluorinated and chlorinated;
in formula (6)', X is a hydroxy group, an alkoxy group, an oxycarbonyl group, an oxysulfonyl group, an oxycarboxy group, or a hydrogen atom; and
in formula (6)', n is a natural number of 1 to 3.

Embodiment (19) of the present disclosure is a method for producing a fluorine-containing polymerizable monomer represented by the following formula (6)', the method including: a step of providing a compound represented by the following formula (10); and a step of reacting the compound represented by formula (10) with a compound represented by the following formula (11): 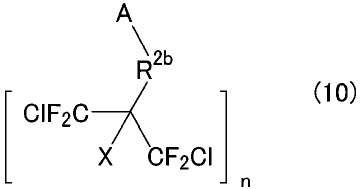
wherein, in formula (10), R^{2b} is an alkylene group optionally having a linear, branched, or cyclic structure, an aromatic ring, an ester, a carbonyl, an ether, an amide, an amine, or a composite substituent thereof, a part of which is optionally fluorinated, chlorinated, or both fluorinated and chlorinated;
in formula (10), X is a hydroxy group, an alkoxy group, an oxycarbonyl group, an oxysulfonyl group, an oxycarboxy group, or a hydrogen atom;
in formula (10), A is a leaving group; and
in formula (10), n is a natural number of 1 to 3, 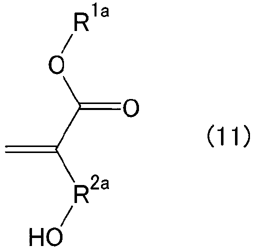
wherein, in formula (11), R^{1a} is a C1-C10 linear or C3-C10 branched alkyl group, some or all of hydrogen atoms bonded to carbon atoms in the alkyl group being optionally replaced by fluorine atoms, chlorine atoms, or both; and
in formula (11), R^{2a} is an alkylene group optionally having a linear, branched, or cyclic structure, an aromatic ring, an ester, a carbonyl, an ether, an amide, an amine, or a composite substituent thereof, a part of which is optionally fluorinated, chlorinated, or both fluorinated and chlorinated, 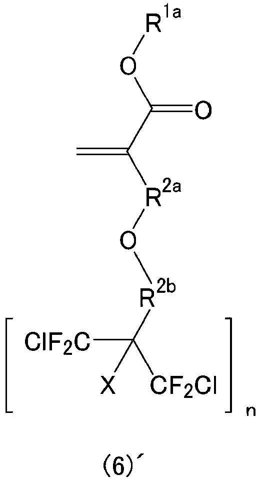
wherein, in formula (6)', R^{1a} is a C1-C10 linear or C3-C10 branched alkyl group, some or all of hydrogen atoms bonded to carbon atoms in the alkyl group being optionally replaced by fluorine atoms, chlorine atoms, or both;
in formula (6)', R^{2a} is an alkylene group optionally having a linear, branched, or cyclic structure, an aromatic ring, an ester, a carbonyl, an ether, an amide, an amine, or a composite substituent thereof, a part of which is optionally fluorinated, chlorinated, or both fluorinated and chlorinated;
in formula (6)', R^{2b} is an alkylene group optionally having a linear, branched, or cyclic structure, an aromatic ring, an ester, a carbonyl, an ether, an amide, an amine, or a composite substituent thereof, a part of which is optionally fluorinated, chlorinated, or both fluorinated and chlorinated;
in formula (6)', X is a hydroxy group, an alkoxy group, an oxycarbonyl group, an oxysulfonyl group, an oxycarboxy group, or a hydrogen atom; and in formula (6)', n is a natural number of 1 to 3.

### - Advantageous Effects of Invention

The fluorine-containing polymer of the present disclosure has water repellency and photosensitivity equivalent to those of conventional fluorine-containing polymers but can be decomposed at a lower temperature. The fluorine-containing polymer of the present disclosure can be used as a resist for photolithography or the like, and the waste material derived from the fluorine-containing polymer of the present disclosure can be disposed of by precipitation, adsorption, activated sludge, or other methods that are simpler than combustion, thereby contributing to energy reduction in semiconductor processes.

### DESCRIPTION OF EMBODIMENTS

Hereinafter, the present disclosure will be described in detail, but the description of the constituent elements described below illustrates embodiments of the present disclosure, and the present disclosure is not limited to these specific contents. Various modifications may be made within the scope of its gist.

In the section of the present specification entitled "DESCRIPTION OF EMBODIMENTS", the items indicated by "[" and "]" or "<" and ">" are merely symbols and do not have any meaning by themselves.

### <Fluorine-Containing Polymerizable Monomer>

First, a fluorine-containing polymerizable monomer according to the present disclosure will be described.

The fluorine-containing polymerizable monomer of the present disclosure is a fluorine-containing polymerizable monomer represented by the following formula (6) or (6)': 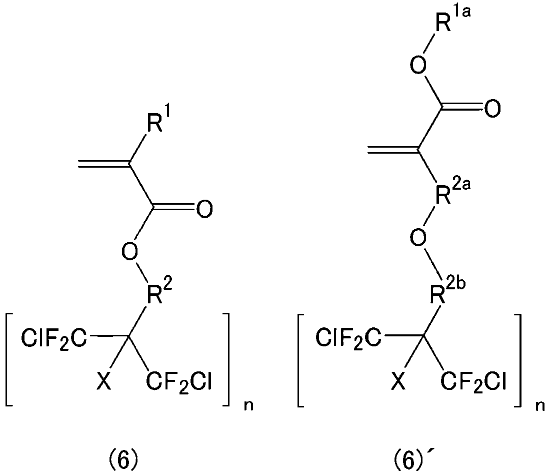
wherein, in formula (6), R¹ is a hydrogen atom, a fluorine atom, a chlorine atom, or a C1-C10 linear or C3-C10 branched alkyl group, some or all of the hydrogen atoms bonded to the carbon atoms in the alkyl group being optionally replaced by fluorine atoms;
in formula (6), R² is a single bond, an alkylene group optionally having a linear, branched, or cyclic structure, an aromatic ring, an ester, a carbonyl, an ether, an amide, an amine, or a composite substituent thereof, a part of which is optionally fluorinated and/or chlorinated;
in formula (6)', R^{1a} is a C1-C10 linear or C3-C10 branched alkyl group, some or all of the hydrogen atoms bonded to the carbon atoms in the alkyl group being optionally replaced by fluorine atoms and/or chlorine atoms;
in formula (6)', R^{2a} is an alkylene group optionally having a linear, branched, or cyclic structure, an aromatic ring, an ester, a carbonyl, an ether, an amide, an amine, or a composite substituent thereof, a part of which is optionally fluorinated and/or chlorinated;
in formula (6)', R^{2b} is an alkylene group optionally having a linear, branched, or cyclic structure, an aromatic ring, an ester, a carbonyl, an ether, an amide, an amine, or a composite substituent thereof, a part of which is optionally fluorinated and/or chlorinated;
in formulae (6) and (6)', X is a hydroxy group, an alkoxy group, an oxycarbonyl group, an oxysulfonyl group, an oxycarboxy group, or a hydrogen atom; and in formulae (6) and (6)', n is a natural number of 1 to 3.

Examples of the alkyl group exemplified as R¹ include a methyl group, an ethyl group, and a propyl group such as a 1-propyl group or a 2-propyl group.

Examples of the alkyl group exemplified as R^{1a} include a methyl group, an ethyl group, and a propyl group such as a 1-propyl group, a 2-propyl group, or a 1,3-dichloro-1,1,3,3-tetrafluoroisopropyl group.

Examples of the alkylene groups exemplified as R², R^{2a}, and R^{2b} include saturated acyclic hydrocarbon groups such as a methylene group, an ethylene group, a propylene group such as a 1,3-propylene group or a 1,2-propylene group, a tetramethylene group, a pentamethylene group, a hexamethylene group, a heptamethylene group, an octamethylene group, a nonamethylene group, a decamethylene group, an undecamethylene group, a dodecamethylene group, a tridecamethylene group, a tetradecamethylene group, a pentadecamethylene group, a hexadecamethylene group, a heptadecamethylene group, an octadecamethylene group, a nonadecamethylene group, an insalene group, a 1-methyl-1,3-propylene group, a 2-methyl-1,3-propylene group, a 2-methyl-1,2-propylene group, a 1-methyl-1,4-butylene group, a 2-methyl-1,4-butylene group, a methylidene group, an ethylidene group, a propylidene group, and a 2-propylidene group; monocyclic hydrocarbon ring groups such as C3-C10 cycloalkylene groups such as a cyclobutylene group such as a 1,3-cyclobutylene group, a cyclopentylene group such as a 1,3-cyclopentylene group, a cyclohexylene group such as a 1,4-cyclohexylene group, and a cyclooctylene group such as a 1,5-cyclooctylene group; and bridged cyclic hydrocarbon ring groups such as bi- to tetracyclic hydrocarbon ring groups such as a norbornylene group such as a 1,4-norbornylene group or a 2,5-norbornylene group, and an adamantylene group such as a 1,5-adamantylene group or a 2,6-adamantylene group.

The amides exemplified as R², R^{2a}, and R^{2b} have a structure represented by - C(=O)NB¹-, wherein B¹ represents a hydrogen atom or a monovalent organic group.

The ethers exemplified as R², R^{2a}, and R^{2b} have a structure represented by -O-.

The amines exemplified as R², R^{2a}, and R^{2b} have a structure represented by - NB²-, wherein B² represents a hydrogen atom or a monovalent organic group.

In formula (6)', R^{2a} and R^{2b} may be the same as or different from each other.

In formulae (6) and (6)', X is a hydroxy group, an alkoxy group, an oxycarbonyl group, an oxysulfonyl group, an oxycarboxy group, or a hydrogen atom. Among these, X is preferably a hydroxy group or a hydrogen atom.

In the case where X is a hydroxy group, it tends to be possible to more suitably provide a polymer that can have properties such as water repellency, adhesion, and alkali solubility. Also, in the case where X is an alkoxy group, an oxycarbonyl group, an oxysulfonyl group, or an oxycarboxy group, it tends to be possible to more suitably provide a polymer that can have properties such as water repellency, water resistance, and polarity conversion by an acid or the like. Also, in the case where X is a hydrogen atom, it tends to be possible to more suitably provide a polymer that can have properties such as water repellency and water resistance.

The oxycarbonyl group as X is a group represented by -O-C(=O)B³, wherein B³ represents a hydrogen atom or a monovalent organic group, and examples include - O-C(=O)CH₃.

The monovalent organic group for B³ refers to a group having one or more carbon atoms, preferably 1 to 30 carbon atoms, more preferably 1 to 8 carbon atoms, still more preferably 1 to 3 carbon atoms, particularly preferably 1 to 2 carbon atoms.

Examples of such monovalent organic groups include monovalent hydrocarbon groups.

The monovalent organic group (preferably monovalent hydrocarbon group) for B³ may have a heteroatom. Examples of the heteroatom include a nitrogen atom, an oxygen atom, a sulfur atom, halogen atoms, a phosphorus atom, and a silicon atom. The monovalent organic group (preferably monovalent hydrocarbon group) for B³ may have a plurality of such heteroatoms.

Examples of monovalent hydrocarbon groups include alkyl groups, alkenyl groups, alkynyl groups, alkylidene groups, aryl groups, aralkyl groups, cycloalkyl groups, and heterocyclic groups. Among these, alkyl groups are preferable.

The alkyl groups may be, for example, linear or branched, and examples include a methyl group, an ethyl group, a propyl group, a butyl group, a pentyl group, a hexyl group, a heptyl group, an octyl group, a nonyl group, and a decyl group. It should be noted that, since the alkyl groups may be linear or branched, the propyl group refers to a n-propyl group and an isopropyl group, the butyl group refers to a n-butyl group, a sec-butyl group, and a tert-butyl group, and the same applies to other groups that may be linear or branched.

The alkenyl groups may be, for example, linear or branched, and examples include a vinyl group, an allyl group, a propenyl group, and a methylethenyl group.

The alkynyl groups may be, for example, linear or branched, and examples include an ethynyl group and a propargyl group.

The alkylidene groups may be, for example, linear or branched, and examples include a methylidene group and an ethylidene group.

Examples of the aryl groups include a phenyl group, a tolyl group, a xylyl group, and a naphthyl group.

Examples of the aralkyl groups include a benzyl group and a phenethyl group.

Examples of the cycloalkyl groups include an adamantyl group, a cyclopentyl group, a cyclohexyl group, and a cyclooctyl group.

Examples of the heterocyclic groups include an epoxy group and an oxetanyl group.

Examples of the halogen atom that may be contained in the monovalent organic group for B³ include a fluorine atom, a chlorine atom, a bromine atom, and an iodine atom.

The oxysulfonyl group as X is a group represented by -O-SO₂-B³, wherein B³ represents a hydrogen atom or a monovalent organic group, and examples include -O-SO₂-CH₃.

The oxycarboxy group as X is a group represented by -O-C(=O)-O-B³, wherein B³ represents a hydrogen atom or a monovalent organic group, and examples include - O-C(=O)-O-CH₃ and -O-C(=O)-O-C(CH₃)₃.

The fluorine-containing polymerizable monomer represented by formula (6) can be produced by, for example, the following method.

A method for producing the fluorine-containing polymerizable monomer represented by formula (6) includes: a step of providing a compound represented by the following formula (7); and a step of reacting the compound represented by formula (7) with at least one selected from the group consisting of an acid halide, an acid anhydride, an ester, and a carboxylic acid: 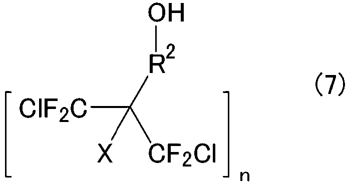
wherein, in formula (7), R² is a single bond, an alkylene group optionally having a linear, branched, or cyclic structure, an aromatic ring, an ester, a carbonyl, an ether, an amide, an amine, or a composite substituent thereof, a part of which is optionally fluorinated and/or chlorinated;
X is a hydroxy group, an alkoxy group, an oxycarbonyl group, an oxysulfonyl group, an oxycarboxy group, or a hydrogen atom; and
n is a natural number of 1 to 3.

The compound represented by formula (7) is preferably other than compounds in which R² is a single bond and X is hydrogen.

The compound represented by formula (7) can be synthesized by a known organic synthesis method.

Examples of the acid halide include methacryloyl chloride, methacryloyl bromide, methacryloyl fluoride, acryloyl chloride, acryloyl bromide, and acryloyl fluoride. Examples of the acid anhydride include methacrylic anhydride and acrylic anhydride. Examples of the ester include methyl methacrylate, ethyl methacrylate, methyl acrylate, butyl acrylate, n-propyl acrylate, iso-propyl acrylate, n-butyl acrylate, iso-butyl acrylate, sec-butyl acrylate, tert-butyl acrylate, n-propyl methacrylate, iso-propyl methacrylate, n-butyl methacrylate, iso-butyl methacrylate, sec-butyl methacrylate, and tert-butyl methacrylate. Examples of the carboxylic acid include methacrylic acid and acrylic acid.

The reaction between the compound represented by formula (7) and any of these compounds may be carried out with the addition of an acid or a base, as necessary. Also, the reaction conditions are preferably 0°C to 130°C for 0.5 to 10 hours.

The separation and purification of the product after the reaction may be carried out by a conventional method. For example, concentration, distillation, extraction, recrystallization, filtration, column chromatography, and other methods can be used, and two or more methods may be used in combination.

The fluorine-containing polymerizable monomer represented by formula (6)' can be produced by, for example, the following method.

A method for producing the fluorine-containing polymerizable monomer represented by formula (6)' includes: a step of providing a compound represented by the following formula (8); and a step of reacting the compound represented by formula (8) with a compound represented by the following formula (9): 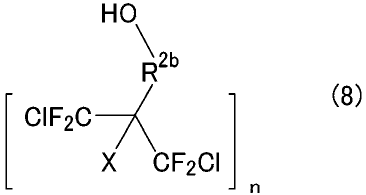
wherein, in formula (8), R^{2b} is an alkylene group optionally having a linear, branched, or cyclic structure, an aromatic ring, an ester, a carbonyl, an ether, an amide, an amine, or a composite substituent thereof, a part of which is optionally fluorinated and/or chlorinated;
in formula (8), X is a hydroxy group, an alkoxy group, an oxycarbonyl group, an oxysulfonyl group, an oxycarboxy group, or a hydrogen atom; and in formula (8), n is a natural number of 1 to 3, 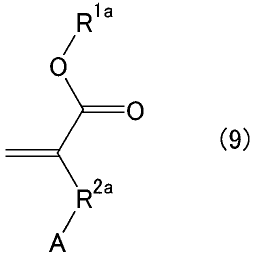
wherein, in formula (9), R^{1a} is a C1-C10 linear or C3-C10 branched alkyl group, some or all of the hydrogen atoms bonded to the carbon atoms in the alkyl group being optionally replaced by fluorine atoms and/or chlorine atoms;
in formula (9), R^{2a} is an alkylene group optionally having a linear, branched, or cyclic structure, an aromatic ring, an ester, a carbonyl, an ether, an amide, an amine, or a composite substituent thereof, a part of which is optionally fluorinated and/or chlorinated; and
in formula (9), A is a leaving group.

The fluorine-containing polymerizable monomer represented by formula (6)' can also be produced by, for example, the following method.

A method for producing the fluorine-containing polymerizable monomer represented by formula (6)' includes: a step of providing a compound represented by the following formula (10); and a step of reacting the compound represented by formula (10) with a compound represented by the following formula (11): 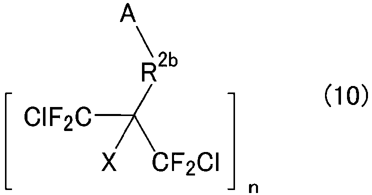
wherein, in formula (10), R^{2b} is an alkylene group optionally having a linear, branched, or cyclic structure, an aromatic ring, an ester, a carbonyl, an ether, an amide, an amine, or a composite substituent thereof, a part of which is optionally fluorinated and/or chlorinated;
in formula (10), X is a hydroxy group, an alkoxy group, an oxycarbonyl group, an oxysulfonyl group, an oxycarboxy group, or a hydrogen atom;
in formula (10), A is a leaving group; and
in formula (10), n is a natural number of 1 to 3, 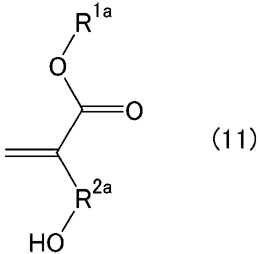
wherein, in formula (11), R^{1a} is a C1-C10 linear or C3-C10 branched alkyl group, some or all of the hydrogen atoms bonded to the carbon atoms in the alkyl group being optionally replaced by fluorine atoms and/or chlorine atoms; and
in formula (11), R^{2a} is an alkylene group optionally having a linear, branched, or cyclic structure, an aromatic ring, an ester, a carbonyl, an ether, an amide, an amine, or a composite substituent thereof, a part of which is optionally fluorinated and/or chlorinated.

The compounds represented by formulae (8) and (10) can be synthesized by known organic synthesis methods.

In formulae (9) and (10), the leaving group exemplified as A is not limited, and may be a halogen atom, a tosyl group, a mesyl group, a triflate group, or the like.

The reaction between the compound represented by formula (8) and the compound represented by formula (9) may be carried out with the addition of a base, as necessary. Also, the reaction conditions are preferably 0°C to 130°C for 0.5 to 10 hours.

The same applies to the reaction conditions for the compound represented by formula (10) and the compound represented by formula (11).

The separation and purification of the product after the reaction may be carried out by a conventional method. For example, concentration, distillation, extraction, recrystallization, filtration, column chromatography, and other methods can be used, and two or more methods may be used in combination.

### <Fluorine-Containing Polymer>

Next, a fluorine-containing polymer according to the present disclosure, which is formed by polymerizing the fluorine-containing polymerizable monomer represented by formula (6) or (6)', will be described.

The fluorine-containing polymer of the present disclosure is a fluorine-containing polymer containing a crosslinking unit represented by the following formula (1) or (1)': 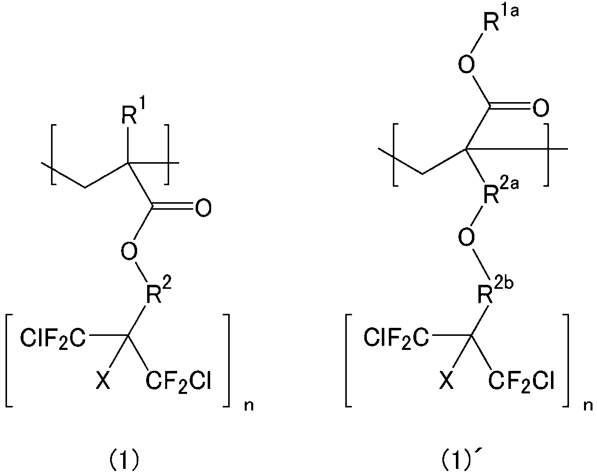
wherein, in formula (1), R¹ is a hydrogen atom, a fluorine atom, a chlorine atom, or a C1-C10 linear or C3-C10 branched alkyl group, some or all of the hydrogen atoms bonded to the carbon atoms in the alkyl group being optionally replaced by fluorine atoms;
in formula (1), R² is a single bond, an alkylene group optionally having a linear, branched, or cyclic structure, an aromatic ring, an ester, a carbonyl, an ether, an amide, an amine, or a composite substituent thereof, a part of which is optionally fluorinated and/or chlorinated;
in formula (1)', R^{1a} is a C1-C10 linear or C3-C10 branched alkyl group, some or all of the hydrogen atoms bonded to the carbon atoms in the alkyl group being optionally replaced by fluorine atoms and/or chlorine atoms;
in formula (1)', R^{2a} is an alkylene group optionally having a linear, branched, or cyclic structure, an aromatic ring, an ester, a carbonyl, an ether, an amide, an amine, or a composite substituent thereof, a part of which is optionally fluorinated and/or chlorinated;
in formula (1)', R^{2b} is an alkylene group optionally having a linear, branched, or cyclic structure, an aromatic ring, an ester, a carbonyl, an ether, an amide, an amine, or a composite substituent thereof, a part of which is optionally fluorinated and/or chlorinated; and
in formulae (1) and (1)', X is a hydroxy group, an alkoxy group, an oxycarbonyl group, an oxysulfonyl group, an oxycarboxy group, or a hydrogen atom.

The fluorine-containing polymers containing the repeating units represented by formulae (1) and (1)' can be obtained by a polymerization step including polymerizing the polymerizable monomers represented by formulae (6) and (6)', respectively.

The fluorine-containing polymer of the present disclosure may contain both the repeating units represented by formulae (1) and (1)'.

It should be noted that, in the following description, the polymerizable monomers represented by formulae (6) and (6)' may also be referred to as "first polymerizable monomers", and the repeating units represented by formulae (1) and (1)' may also be referred to as "first repeating units".

The fluorine-containing polymer may contain at least one second repeating unit other than the repeating units represented by formulae (1) and (1)'. In this case, the second repeating unit may include, for example, at least one repeating unit derived from any of the following polymerizable monomers (second polymerizable monomers).

Examples of the second polymerizable monomers include monomers having a hexafluoroisopropanol group (-C(CF₃)₂OH), acrylic acid esters, methacrylic acid esters, fluorine-containing acrylic acid esters, fluorine-containing methacrylic acid esters, styrenes, fluorine-containing styrenes, vinyl ethers, fluorine-containing vinyl ethers, allyl ethers, fluorine-containing allyl ethers, unsaturated amides, olefins, fluorine-containing olefins, norbornene compounds, fluorine-containing norbornene compounds, vinylsilanes, vinylsulfonic acid or vinylsulfonic acid esters acrylic acid, methacrylic acid maleic acid, maleic anhydride, fumaric acid, sulfur dioxide, and monomers having a lactone structure.

The fluorine-containing polymer of formula (1) or (1)' may also contain, as a third repeating unit, a repeating unit containing an acid-decomposable group.

As for the fluorine-containing polymer containing the third repeating unit, when the fluorine-containing polymer is used as a resist, exposing the resist film formed on a substrate to electromagnetic waves or high-energy rays (such as electron beams) having a wavelength of 300 nm or less causes the acid-decomposable group to decompose, generating an acid in the resist film. This acid can improve the solubility of the exposed portion of the resist film in an alkaline developer during development.

Such fluorine-containing polymers may be homopolymers composed only of the first repeating units, or heteropolymers containing the second and/or third repeating units.

In the case of a fluorine-containing polymer heteropolymer, when all repeating units in the fluorine-containing polymer are taken as 100 mol%, the first repeating unit content may be 22 mol% or more and 99 mol% or less, may be 30 mol% or more and 95 mol% or less, or even may be 40 mol% or more and 90 mol% or less.

The fluorine-containing polymer can be obtained by the polymerization reaction of the polymerizable monomers.

The polymerization reaction is not limited, and may be a radical polymerization reaction, an ionic polymerization reaction, a coordination anionic polymerization reaction, a living anionic polymerization reaction, or a cationic polymerization reaction. Among these, a radical polymerization reaction is preferable.

In the case where the polymerization reaction is a radical polymerization reaction, the polymerization initiator used is not limited as long as it can initiate the polymerization reaction, and azo compounds, peroxide compounds, and redox compounds can be used.

Examples of the azo compounds include azobisisobutyronitrile. Examples of the peroxide compounds include t-butyl peroxypivalate, di-t-butyl peroxide, i-butyryl peroxide, lauroyl peroxide, succinic acid peroxide, dicinnamyl peroxide, di-n-propyl peroxydicarbonate, t-butyl peroxy allyl monocarbonate, benzoyl peroxide, hydrogen peroxide, and ammonium persulfate.

Examples of the redox compounds include combinations of oxidizing agents and reducing agents, in which the oxidizing agents include hydrogen peroxide, persulfates, cumene hydroperoxide, etc., while the reducing agents include iron(II) ion salts, copper(I) ion salts, ammonia, triethylamine, etc.

In the radical polymerization reaction, a polymerization solvent may also be used.

Any polymerization solvent that does not inhibit the radical polymerization reaction can be used, and it may be either an organic solvent or water. Examples of the organic solvent include hydrocarbon solvents, ester solvents, ketone solvents, alcohol solvents, ether solvents, cyclic ether solvents, fluorocarbon solvents, and aromatic solvents. Only one of these solvents may be used, or two or more thereof may be used in combination.

Examples of the ester solvents include acetic acid and n-butyl acetate.

Examples of the ketone solvents include acetone and methyl isobutyl ketone.

Examples of the hydrocarbon solvents include toluene and cyclohexane.

Examples of the alcohol solvents include methanol, isopropyl alcohol, and ethylene glycol monomethyl ether.

In the radical polymerization reaction, a molecular weight modifier such as mercaptan may also be used.

Although the reaction temperature in the radical polymerization reaction may be varied as appropriate depending on the radical polymerization initiator or the type of radical polymerization initiator, it is preferably 20°C or higher and 200°C or lower, more preferably 30°C or higher and 140°C or lower.

Following the polymerization step, a known method can be used to remove the medium, either organic solvent or water, from the solution or dispersion containing the synthesized fluorine-containing polymer. Specific examples of such methods include reprecipitation, filtration, and heating distillation under reduced pressure.

Fluorine-containing polymers containing the repeating units represented by formulae (1) and (1)' can be produced through the above steps. The weight average molecular weight of the fluorine-containing polymer of the present disclosure may be 5000 to 20000, or even 7000 to 12000. Moreover, in the present specification, the weight average molecular weight of the fluorine-containing polymer means the numerical value measured by gel permeation chromatography (GPC) under the following conditions.

### [GPC Conditions]

Apparatus: HLC-8320GPC manufactured by Tosoh Corporation
Columns for polymerizable monomer analysis: TSKgel series manufactured by Tosoh Corporation (G2500HXL, G2000HXL, G1000HXL, and G1000HXL connected in series in that order)
Columns for polymer analysis: TSKgel series manufactured by Tosoh Corporation (G2500HXL, G2000HXL, G1000HXL, and G1000HXL connected in series in that order)
Temperature program: 40°C (retention)
Flow rate: 1 mL/min
Detector: differential refractometer (RI)
Eluent: tetrahydrofuran (THF)
Reference materials: polystyrene standard solutions

### <Specific Examples of Fluorine-Containing Polymer>

In the fluorine-containing polymer of the present disclosure, the repeating unit represented by formula (1) may include a repeating unit represented by the following formula (2). It should be noted that the fluorine-containing polymer of the present disclosure may be composed only of the repeating unit represented by the following formula (2).

Also, in the fluorine-containing polymer of the present disclosure, the repeating unit represented by formula (1)' may include a repeating unit represented by the following formula (2)'. It should be noted that the fluorine-containing polymer of the present disclosure may be composed only of the repeating unit represented by the following formula (2)'.

Furthermore, the fluorine-containing polymer of the present disclosure may contain both the repeating unit represented by the following formula (2) and the repeating unit represented by the following formula (2)'. In the case where the fluorine-containing polymer of the present disclosure contains the repeating units represented by the following formulae (2) and (2)', since the terminal of the side chain is a hydroxy group, the fluorine-containing polymer can have properties such as water repellency, adhesion, and alkali solubility.

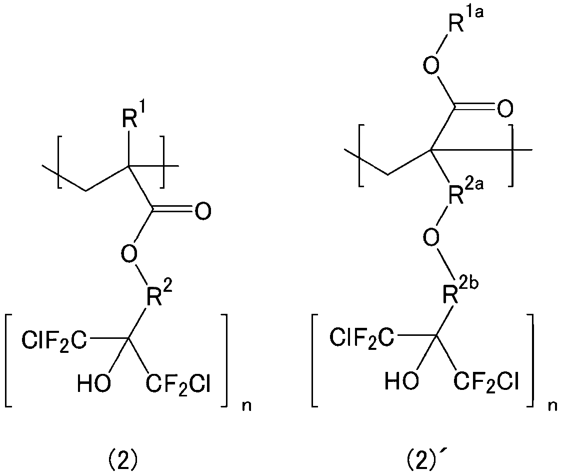

In the fluorine-containing polymer of the present disclosure, in which the repeating units represented by formulae (1) and (1)' include the repeating units represented by formulae (2) and (2)', respectively, the fluorine-containing polymer of the present disclosure may contain a repeating unit represented by the following formula (3).

Since the fluorine-containing polymer according to this embodiment can have properties such as alkali solubility, it is suited for a resin contained in a resist upper layer film, for example. In particular, it is preferably for use in a resist upper layer film used in a liquid immersion lithography process. 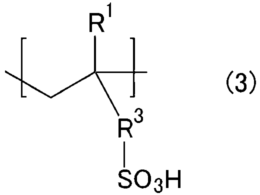 wherein R³ is a single bond, an alkylene group optionally having a linear, branched, or cyclic structure, an aromatic ring, an ester, a carbonyl, an ether, an amide, an amine, or a composite substituent thereof, a part of which is optionally fluorinated and/or chlorinated.

Examples of the alkylene group exemplified as R³ include saturated acyclic hydrocarbon groups such as a methylene group, an ethylene group, a propylene group such as a 1,3-propylene group or a 1,2-propylene group, a tetramethylene group, a pentamethylene group, a hexamethylene group, a heptamethylene group, an octamethylene group, a nonamethylene group, a decamethylene group, an undecamethylene group, a dodecamethylene group, a tridecamethylene group, a tetradecamethylene group, a pentadecamethylene group, a hexadecamethylene group, a heptadecamethylene group, an octadecamethylene group, a nonadecamethylene group, an insalene group, a 1-methyl-1,3-propylene group, a 2-methyl-1,3-propylene group, a 2-methyl-1,2-propylene group, a 1-methyl-1,4-butylene group, a 2-methyl-1,4-butylene group, a methylidene group, an ethylidene group, a propylidene group, and a 2-propylidene group; monocyclic hydrocarbon ring groups such as C3-C10 cycloalkylene groups such as a cyclobutylene group such as a 1,3-cyclobutylene group, a cyclopentylene group such as a 1,3-cyclopentylene group, a cyclohexylene group such as a 1,4-cyclohexylene group, and a cyclooctylene group such as a 1,5-cyclooctylene group; and bridged cyclic hydrocarbon ring groups such as bi- to tetracyclic hydrocarbon ring groups such as a norbornylene group such as a 1,4-norbornylene group or a 2,5-norbornylene group, and an adamantylene group such as a 1,5-adamantylene group or a 2,6-adamantylene group.

Examples of the aromatic ring exemplified as R³ include an o-phenylene group, a m-phenylene group, and a p-phenylene group.

In the fluorine-containing polymer of the present disclosure, the repeating unit represented by formula (1) may include a repeating unit represented by the following formula (4). It should be noted that the fluorine-containing polymer of the present disclosure may be composed only of the repeating unit represented by the following formula (4).

In the fluorine-containing polymer of the present disclosure, the repeating unit represented by formula (1)' may include a repeating unit represented by the following formula (4)'. It should be noted that the fluorine-containing polymer of the present disclosure may be composed only of the repeating unit represented by the following formula (4)'.

Furthermore, the fluorine-containing polymer of the present disclosure may contain both the repeating unit represented by the following formula (4) and the repeating unit represented by the following formula (4)'. In the case where the fluorine-containing polymer of the present disclosure contains the repeating units represented by the following formulae (4) and (4)', since the terminal of the side chain is a hydrogen group, the fluorine-containing polymer can have properties such as water repellency and water resistance.

Also, in the case where the fluorine-containing polymer of the present disclosure contains the repeating units represented by the following formulae (4) and (4)', it is suited for a resin contained in a resist upper layer film, for example. In particular, it is preferably for use in a resist upper layer film used in a liquid immersion lithography process.

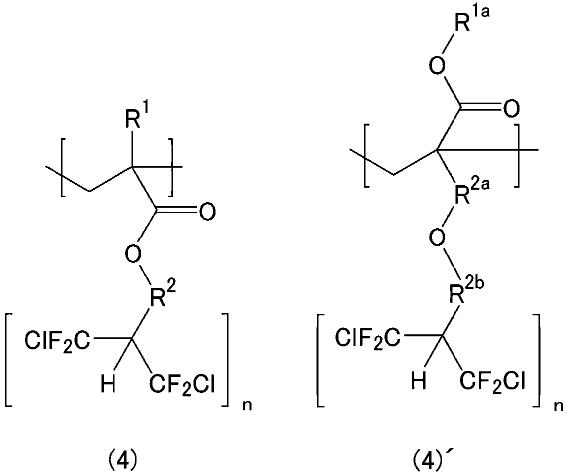

In the case where the fluorine-containing polymer of the present disclosure contains the repeating units represented by formulae (4) and (4)', the fluorine-containing polymer of the present disclosure may contain the repeating units represented by formulae (2) and (2)'.

Since, in the fluorine-containing polymer according to this embodiment, the terminals of the side chains in the repeating units represented by formulae (2) and (2)' are hydroxy groups, the fluorine-containing polymer can have properties such as water repellency, adhesion, and alkali solubility.

In the case where the fluorine-containing polymer of the present disclosure contains the repeating units represented by formulae (4) and (4)', the fluorine-containing polymer of the present disclosure may contain a repeating unit represented by the following formula (5).

Since the fluorine-containing polymer according to this embodiment can have properties such as water repellency, water resistance, removal of acid-dissociable groups by an acid generated during exposure, and resultant alkali solubility, it is suited for a resin contained in an additive for a resist film, for example. 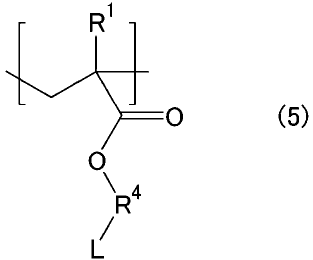
wherein R⁴ is a single bond, an alkylene group optionally having a linear, branched, or cyclic structure, an aromatic ring, an ester, a carbonyl, an ether, an amide, an amine, or a composite substituent thereof, a part of which is optionally fluorinated and/or chlorinated; and
L is an acid-dissociable group.

Examples of the alkylene group include saturated acyclic hydrocarbon groups such as a methylene group, an ethylene group, a propylene group such as a 1,3-propylene group or a 1,2-propylene group, a tetramethylene group, a pentamethylene group, a hexamethylene group, a heptamethylene group, an octamethylene group, a nonamethylene group, a decamethylene group, an undecamethylene group, a dodecamethylene group, a tridecamethylene group, a tetradecamethylene group, a pentadecamethylene group, a hexadecamethylene group, a heptadecamethylene group, an octadecamethylene group, a nonadecamethylene group, an insalene group, a 1-methyl-1,3-propylene group, a 2-methyl-1,3-propylene group, a 2-methyl-1,2-propylene group, a 1-methyl-1,4-butylene group, a 2-methyl-1,4-butylene group, a methylidene group, an ethylidene group, a propylidene group, and a 2-propylidene group; monocyclic hydrocarbon ring groups such as C3-C10 cycloalkylene groups such as a cyclobutylene group such as a 1,3-cyclobutylene group, a cyclopentylene group such as a 1,3-cyclopentylene group, a cyclohexylene group such as a 1,4-cyclohexylene group, and a cyclooctylene group such as a 1,5-cyclooctylene group; and bridged cyclic hydrocarbon ring groups such as bi- to tetracyclic hydrocarbon ring groups such as a norbornylene group such as a 1,4-norbornylene group or a 2,5-norbornylene group, and an adamantylene group such as a 1,5-adamantylene group or a 2,6-adamantylene group.

Examples of the aromatic ring include an o-phenylene group, a m-phenylene group, and a p-phenylene group.

Examples of the acid-dissociable group include a tert-butyl group, a tert-amyl group, a 1,1-dimethylpropyl group, a 1-ethyl-1-methylpropyl group, a 1,1-dimethylbutyl group, an allyl group, a 1-pyrenylmethyl group, a 5-dibenzosuberyl group, a triphenylmethyl group, a 1-ethyl-1-methylbutyl group, a 1,1-diethylpropyl group, a 1,1-dimethyl-1-phenylmethyl group, a 1-methyl-1-ethyl-1-phenylmethyl group, a 1,1-diethyl-1-phenylmethyl group, a 1-methylcyclohexyl group, a 1-ethylcyclohexyl group, a 1-methylcyclopentyl group, a 1-ethylcyclopentyl group, a 1-isobornyl group, a 1-methyladamantyl group, a 1-ethyladamantyl group, a 1-isopropyladamantyl group, a 1-isopropylnorbornyl group, a 1-isopropyl-(4-methylcyclohexyl) group, a tert-butoxycarbonyl group, a tert-amyloxycarbonyl group, a methoxycarbonyl group, an ethoxycarbonyl group, an i-propoxycarbonyl group, a methoxymethyl group, an ethoxyethyl group, a butoxyethyl group, a cyclohexyloxyethyl group, a benzyloxyethyl group, a phenethyloxyethyl group, an ethoxypropyl group, a benzyloxypropyl group, a phenethyloxypropyl group, an ethoxybutyl group, an ethoxyisobutyl group, a trimethylsilyl group, an ethyldimethylsilyl group, a methyldiethylsilyl group, a triethylsilyl group, an i-propyldimethylsilyl group, a methyldi-i-propylsilyl group, a tri-i-propylsilyl group, a tert-butyldimethylsilyl group, a methyldi-tert-butylsilyl group, a tri-tert-butylsilyl group, a phenyldimethylsilyl group, a methyldiphenylsilyl group, a triphenylsilyl group, an acetyl group, a propionyl group, a butyryl group, a heptanoyl group, a hexanoyl group, a valeryl group, a pivaloyl group, an isovaleryl group, a lauroyl group, a myristoyl group, a palmitoyl group, a stearoyl group, an oxalyl group, a malonyl group, a succinyl group, a glutaryl group, an adipoyl group, a piperoyl group, a suberoyl group, an azelaoyl group, a sebacoyl group, a (meth)acryloyl group, a propioloyl group, a crotonoyl group, an oleoyl group, a maleoyl group, a fumaroyl group, a mesaconoyl group, a camphoroyl group, a benzoyl group, a phthaloyl group, an isophthaloyl group, a terephthaloyl group, a naphthoyl group, a toluoyl group, a hydroatropoyl group, an atropoyl group, a cinnamoyl group, a furoyl group, a thenoyl group, a nicotinoyl group, and an isonicotinoyl group.

Furthermore, in the fluorine-containing polymer of the present disclosure, the repeating units represented by formulae (1) and/or (1)' may include the repeating units represented by formulae (2) and/or (2)', as well as the repeating units represented by formulae (4) and/or (4)'.

Since the fluorine-containing polymer according to this embodiment can have properties such as adhesion, alkali solubility, water repellency, water resistance, deprotection of acid-dissociable groups by an acid, and resultant alkali solubility of the exposed portion, it is suited for a resin contained in an additive for a resist film, for example.

In the case where the fluorine-containing polymer of the present disclosure contains the repeating units represented by formulae (2) and/or (2)', as well as the repeating units represented by formulae (4) and/or (4)', it may further contain the repeating unit represented by formula (5).

It should be noted that the fluorine-containing polymer of the present disclosure may be composed only of the repeating units represented by formulae (2) and/or (2)', the repeating units represented by formulae (4) and/or (4)', and the repeating unit represented by formula (5).

It should be noted that, in the fluorine-containing polymer of the present disclosure, R¹ in each repeating unit represented by formula (1) may be the same or different.

Similarly, R² in each repeating unit represented by formula (1) may also be the same or different.

Similarly, X in each repeating unit represented by formula (1) may also be the same or different.

Similarly, n in each repeating unit represented by formula (1) may also be the same or different.

Also, in the fluorine-containing polymer of the present disclosure, R^{1a} in each repeating unit represented by formula (1)' may be the same or different.

Similarly, R^{2a} or R^{2b} in each repeating unit represented by formula (1)' may also be the same or different.

Similarly, X in each repeating unit represented by formula (1)' may also be the same or different.

Similarly, n in each repeating unit represented by formula (1)' may also be the same or different.

Also, in the fluorine-containing polymer of the present disclosure, R³ in each repeating unit represented by formula (3) or R⁴ in each repeating unit represented by formula (5) may also be the same or different.

Similarly, L in each repeating unit represented by formula (5) may also be the same or different.

Furthermore, R¹ in the repeating unit represented by formula (1), R¹ in the repeating unit represented by formula (3), and R¹ in the repeating unit represented by formula (5) are common; however, in the case where the fluorine-containing polymer of the present disclosure contains two or more of these repeating units, R¹ in each repeating unit may be the same or different.

Next, a method for using the fluorine-containing polymer of the present disclosure will be described.

### <Formation of Resist Film and Upper Layer Film>

The fluorine-containing polymer of the present disclosure can be used as a component of a resist film and/or an upper layer film for protecting the resist film.

The resist film and the upper layer film can be obtained by applying a photosensitive resin composition containing the fluorine-containing polymer of the present disclosure onto a substrate or the like.

The photosensitive resin composition for forming the resist film is a composition for forming a resist pattern according to the present disclosure, which contains the fluorine-containing polymer of the present disclosure, an acid generator, and a solvent.

The photosensitive resin composition for forming the upper layer film is a composition for forming a resist upper layer film according to the present disclosure, which contains the fluorine-containing polymer of the present disclosure and a solvent.

The resist film may be either of a negative type or a positive type.

The substrate is not limited, and examples include a silicon wafer, a compound semiconductor substrate, and an insulating substrate. The substrate may be one on which an antireflection film has been formed. Also, when forming the upper layer film, the photosensitive resin composition may be applied onto a resist film.

In the case where the fluorine-containing polymer of the present disclosure is used to form a resist film, the composition for forming a resist pattern may be applied onto a substrate to form a resist film. Then, the resist film may be subjected to exposure or liquid immersion lithography. Also, in the case where the fluorine-containing polymer of the present disclosure is used to form an upper layer film, the composition for forming a resist upper layer film may be applied onto a resist film and/or a resist pattern to form an upper layer film. Then, the upper layer film may be subjected to exposure or liquid immersion lithography.

### <<Composition for Forming Resist Pattern>>

As described above, in the case where the fluorine-containing polymer of the present disclosure is used to form a resist film, the composition for forming a resist pattern may contain the fluorine-containing polymer of the present disclosure, an acid generator, and a solvent.

Examples of the acid generator include onium salt acid generators such as iodonium salts and sulfonium salts, oxime sulfonate acid generators, diazomethane acid generators such as bisalkyl- or bisaryl-sulfonyl diazomethanes and poly(bissulfonyl) diazomethanes, nitrobenzyl sulfonate acid generators, iminosulfonate acid generators, and disulfone acid generators.

Specific examples of the onium salt acid generators include diphenyliodonium trifluoromethanesulfonate or nonafluorobutanesulfonate, bis(4-tert-butylphenyl)iodonium trifluoromethanesulfonate or nonafluorobutanesulfonate, triphenylsulfonium trifluoromethanesulfonate and the corresponding heptafluoropropanesulfonate or nonafluorobutanesulfonate, tri(4-methylphenyl)sulfonium trifluoromethanesulfonate and the corresponding heptafluoropropanesulfonate or nonafluorobutanesulfonate, dimethyl(4-hydroxynaphthyl)sulfonium trifluoromethanesulfonate and the corresponding heptafluoropropanesulfonate or nonafluorobutanesulfonate, monophenyldimethylsulfonium trifluoromethanesulfonate and the corresponding heptafluoropropanesulfonate or nonafluorobutanesulfonate, diphenylmonomethylsulfonium trifluoromethanesulfonate and the corresponding heptafluoropropanesulfonate or nonafluorobutanesulfonate, (4-methylphenyl)diphenylsulfonium trifluoromethanesulfonate and the corresponding heptafluoropropanesulfonate or nonafluorobutanesulfonate, (4-methoxyphenyl)diphenylsulfonium trifluoromethanesulfonate and the corresponding heptafluoropropanesulfonate or nonafluorobutanesulfonate, tri(4-tert-butyl)phenylsulfonium trifluoromethanesulfonate and the corresponding heptafluoropropanesulfonate or nonafluorobutanesulfonate, diphenyl(1-(4-methoxy)naphthyl)sulfonium trifluoromethanesulfonate and the corresponding heptafluoropropanesulfonate or nonafluorobutanesulfonate, and di(1-naphthyl)phenylsulfonium trifluoromethanesulfonate and the corresponding heptafluoropropanesulfonate or nonafluorobutanesulfonate. In addition, the foregoing onium salts in which the anionic part of the onium salts has been replaced with methanesulfonate, n-propanesulfonate, n-butanesulfonate, or n-octanesulfonate can also be used.

Specific examples of the oxime sulfonate acid generators include α-(p-toluenesulfonyloxyimino)-benzyl cyanide, α-(p-chlorobenzenesulfonyloxyimino)-benzyl cyanide, α-(4-nitrobenzenesulfonyloxyimino)-benzyl cyanide, α-(4-nitro-2-trifluoromethylbenzenesulfonyloxyimino)-benzyl cyanide, α-(benzenesulfonyloxyimino)-4-chlorobenzyl cyanide, α-(benzenesulfonyloxyimino)-2,4-dichlorobenzyl cyanide, α-(benzenesulfonyloxyimino)-2,6-dichlorobenzyl cyanide, α-(benzenesulfonyloxyimino)-4-methoxybenzyl cyanide, α-(2-chlorobenzenesulfonyloxyimino)-4-methoxybenzyl cyanide, α-(benzenesulfonyloxyimino)-thien-2-ylacetonitrile, α-(4-dodecylbenzenesulfonyloxyimino)-benzyl cyanide, α-[(p-toluenesulfonyloxyimino)-4-methoxyphenyl]acetonitrile, α-[(dodecylbenzenesulfonyloxyimino)-4-methoxyphenyl]acetonitrile, α-(tosyloxyimino)-4-thienyl cyanide, α-(methylsulfonyloxyimino)-1-cyclopentenylacetonitrile, α-(methylsulfonyloxyimino)-1-cyclohexenylacetonitrile, α-(methylsulfonyloxyimino)-1-cycloheptenylacetonitrile, α-(methylsulfonyloxyimino)-1-cyclooctenylacetonitrile, α-(trifluoromethylsulfonyloxyimino)-1-cyclopentenylacetonitrile, α-(trifluoromethylsulfonyloxyimino)-cyclohexylacetonitrile, α-(ethylsulfonyloxyimino)-ethylacetonitrile, α-(propylsulfonyloxyimino)-propylacetonitrile, α-(cyclohexylsulfonyloxyimino)-cyclopentylacetonitrile, α-(cyclohexylsulfonyloxyimino)-cyclohexylacetonitrile, α-(cyclohexylsulfonyloxyimino)-1-cyclopentenylacetonitrile, α-(ethylsulfonyloxyimino)-1-cyclopentenylacetonitrile, α-(isopropylsulfonyloxyimino)-1-cyclopentenylacetonitrile, α-(n-butylsulfonyloxyimino)-1-cyclopentenylacetonitrile, α-(ethylsulfonyloxyimino)-1-cyclohexenylacetonitrile, α-(isopropylsulfonyloxyimino)-1-cyclohexenylacetonitrile, α-(n-butylsulfonyloxyimino)-1-cyclohexenylacetonitrile, α-(methylsulfonyloxyimino)-phenylacetonitrile, α-(methylsulfonyloxyimino)-p-methoxyphenylacetonitrile, α-(trifluoromethylsulfonyloxyimino)-phenylacetonitrile, α-(trifluoromethylsulfonyloxyimino)-p-methoxyphenylacetonitrile, α-(ethylsulfonyloxyimino)-p-methoxyphenylacetonitrile, α-(propylsulfonyloxyimino)-p-methylphenylacetonitrile, and α-(methylsulfonyloxyimino)-p-bromophenylacetonitrile.

As for the diazomethane acid generators such as bisalkyl- or bisaryl-sulfonyl diazomethanes and poly(bissulfonyl) diazomethanes, specific examples of the bisalkyl- or bisaryl-sulfonyl diazomethanes include bis(isopropylsulfonyl)diazomethane, bis(p-toluenesulfonyl)diazomethane, bis(1,1-dimethylethylsulfonyl)diazomethane, bis(cyclohexylsulfonyl)diazomethane, and bis(2,4-dimethylphenylsulfonyl)diazomethane.

Examples of the solvent include methanol, ethanol, 1-propanol, isopropanol, n-propanol, 1-butanol, 2-butanol, 2-methyl-2-propanol, 1-pentanol, 2-pentanol, 3-pentanol, n-hexanol, cyclohexanol, 2-methyl-2-butanol, 3-methyl-2-butanol, 2-methyl-1-butanol, 3-methyl-1-butanol, 2-methyl-1-pentanol, 2-methyl-2-pentanol, 2-methyl-3-pentanol, 3-methyl-1-pentanol, 3-methyl-2-pentanol, 3-methyl-3-pentanol, 4-methyl-1-pentanol, 4-methyl-2-pentanol, ethylene glycol, propylene glycol, tetrahydrofuran, dioxane, ethylene glycol monomethyl ether, ethylene glycol monoethyl ether, ethylene glycol dimethyl ether, ethylene glycol diethyl ether, diethylene glycol monomethyl ether, diethylene glycol monoethyl ether, diethylene glycol dimethyl ether, diethylene glycol diethyl ether, diethylene glycol ethyl methyl ether, propylene glycol monomethyl ether, propylene glycol monoethyl ether, ethylene glycol ethyl ether acetate, diethylene glycol ethyl ether acetate, propylene glycol ethyl ether acetate, propylene glycol monomethyl ether acetate, toluene, xylene, acetone, methyl ethyl ketone, methyl isobutyl ketone, cyclohexanone, 4-hydroxy-4-methyl-2-pentanone, diacetone alcohol, ethyl acetate, butyl acetate, ethyl 2-hydroxypropionate, ethyl 2-hydroxy-2-methylpropionate, ethyl 2-hydroxy-2-methylpropionate, ethyl ethoxyacetate, ethyl hydroxyacetate, methyl 2-hydroxy-3-methylbutanoate, methyl 3-methoxypropionate, ethyl 3-methoxypropionate, ethyl 3-ethoxypropionate, methyl 3-ethoxypropionate, and water.

It should be noted that the composition for forming a resist pattern of the present disclosure may contain a basic compound.

Examples of the basic compound include: monoalkylamines such as n-hexylamine, n-heptylamine, n-octylamine, n-nonylamine, and n-decylamine; dialkylamines such as diethylamine, di-n-propylamine, di-n-heptylamine, di-n-octylamine, and dicyclohexylamine; trialkylamines such as trimethylamine, triethylamine, tri-n-propylamine, tri-n-butylamine, tri-n-hexylamine, tri-n-pentylamine, tri-n-heptylamine, tri-n-octylamine, tri-n-nonylamine, tri-n-decanylamine, and tri-n-dodecylamine; alkyl alcohol amines such as diethanolamine, triethanolamine, diisopropanolamine, triisopropanolamine, di-n-octanolamine, and tri-n-octanolamine; and piperidine, piperazine, 1,5-diazabicyclo[4.3.0]-5-nonene, 1,8-diazabicyclo[5.4.0]-7-undecene, hexamethylenetetramine, and 1,4-diazabicyclo[2.2.2]octane.

Moreover, the composition for forming a resist pattern of the present disclosure may contain a base resin other than the fluorine-containing polymer of the present disclosure, or a weak-acid photoacid generator (weak-acid PAG).

Examples of the base resin include resins containing structures represented by the following chemical formulae: 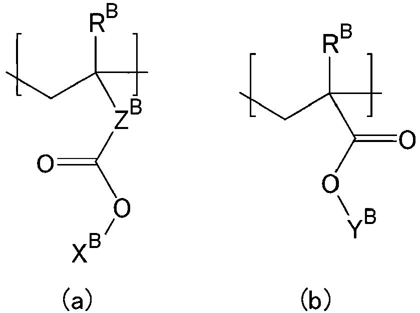 wherein, in formulae (a) and (b), R^{B} is a hydrogen atom, a fluorine atom, a methyl group, or a trifluoromethyl group; Z^{B} is a single bond, a phenylene group, a naphthylene group, or (main chain)-C(=O)-O-Z'-, where Z' is a C1-C10 alkanediyl group optionally containing a hydroxy group, an ether bond, an ester bond, or a lactone ring, or a phenylene group or a naphthylene group; X^{B} is an acid-labile group; and Y^{B} is a hydrogen atom, or a polar group containing at least one structure selected from a hydroxy group, a cyano group, a carbonyl group, a carboxyl group, an ether bond, an ester bond, a sulfonic acid ester bond, a carbonate bond, a lactone ring, a sultone ring, and carboxylic anhydride (-C(=O)-O-C(=O)-).

The alkanediyl group may be linear, branched, or cyclic, and specific examples include a methylene group, an ethane-1,1-diyl group, an ethane-1,2-diyl group, a propane-1,2-diyl group, a propane-2,2-diyl group, a propane-1,3-diyl group, a 2-methylpropane-1,3-diyl group, a butane-1,3-diyl group, a butane-2,3-diyl group, a butane-1,4-diyl group, a pentane-1,3-diyl group, a pentane-1,4-diyl group, a 2,2-dimethylpropane-1,3-diyl group, a pentane-1,5-diyl group, a hexane-1,6-diyl group, a cyclopentane-1,2-diyl group, a cyclopentane-1,3-diyl group, and a cyclohexane-1,6-diyl group.

Examples of structures in which Z^{B} in formula (a) is changed include those shown below, but are not limited thereto. It should be noted that, in the following formulae, R^{B} and X^{B} are the same as described above.

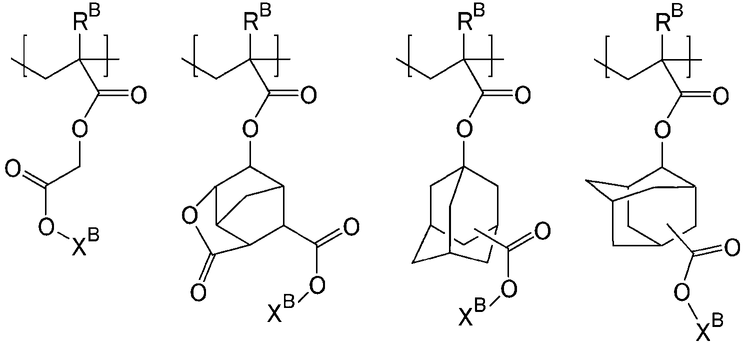

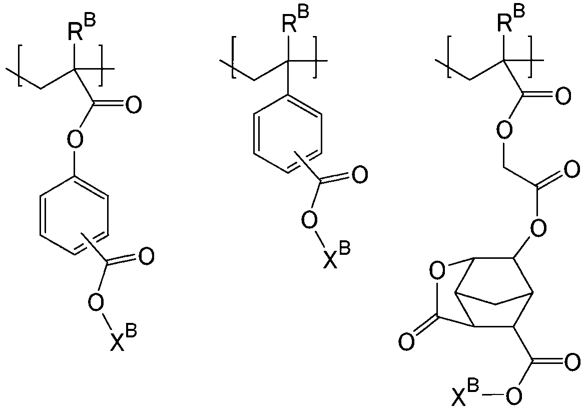

The acid-labile group represented by X^{B} is not limited, and examples include C4-C20 tertiary alkyl groups, trialkylsilyl groups in which each alkyl group is a C1-C6 alkyl group, and C4-C20 oxoalkyl groups.

As the acid-labile group, those represented by the following formulae (xa), (xb), and (xc) are particularly preferable: 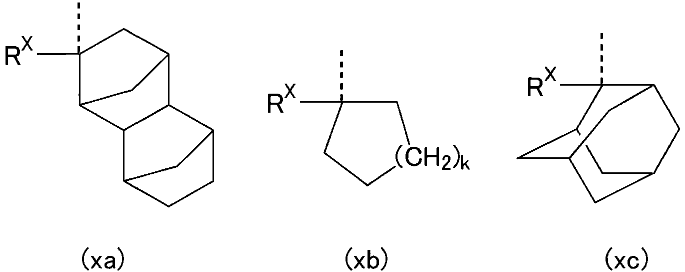 wherein, in formulae (xa), (xb), and (xc), R^{X} is a C1-C10 monovalent hydrocarbon group optionally containing a heteroatom; k is 1 or 2; and the dashed line is a bond.

In the case where the tertiary alicyclic hydrocarbon group represented by formula (xa), (xb), or (xc) is bonded to the ester oxygen, it provides higher acid-decomposability due to steric repulsion than other tertiary alkyl groups, such as a tert-butyl group or a tert-pentyl group.

Examples of the repeating unit represented by formula (a) include those shown below, but are not limited thereto. It should be noted that, in the following formulae, R^{B} is a hydrogen atom, a fluorine atom, a methyl group, or a trifluoromethyl group.

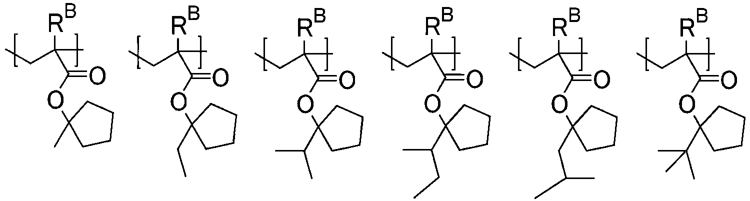

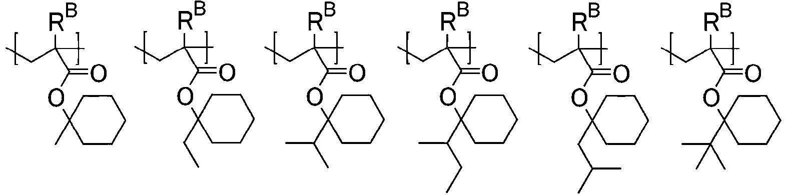

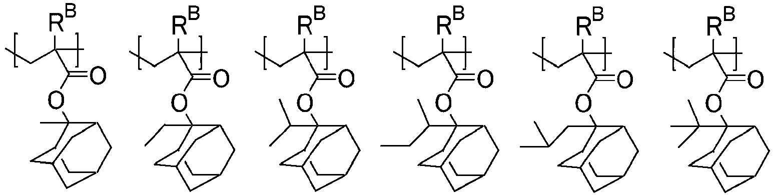

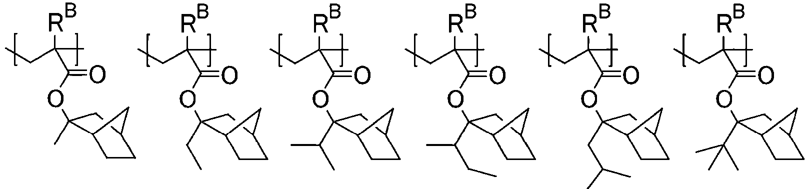

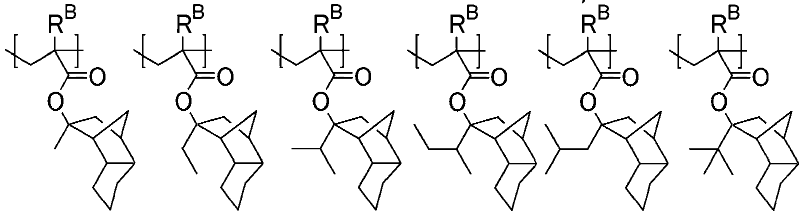

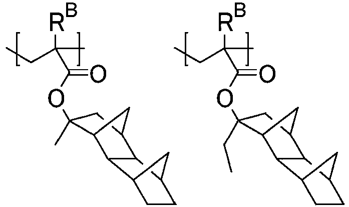

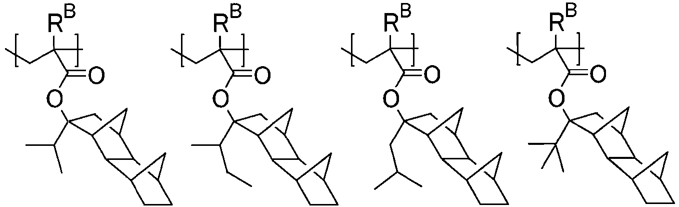

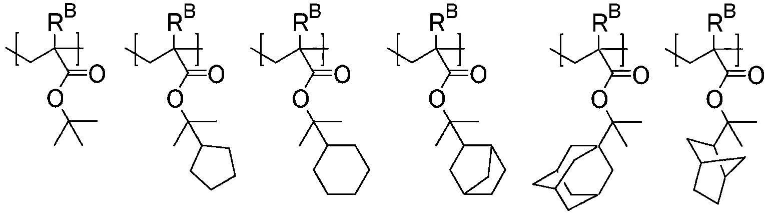

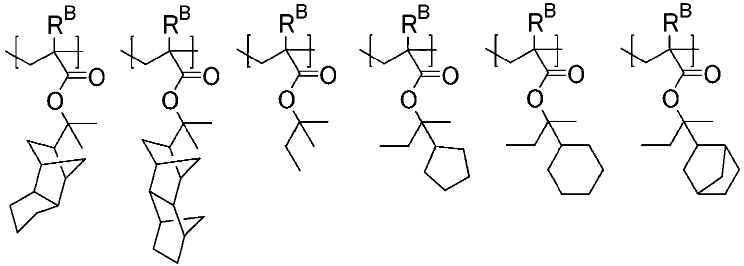

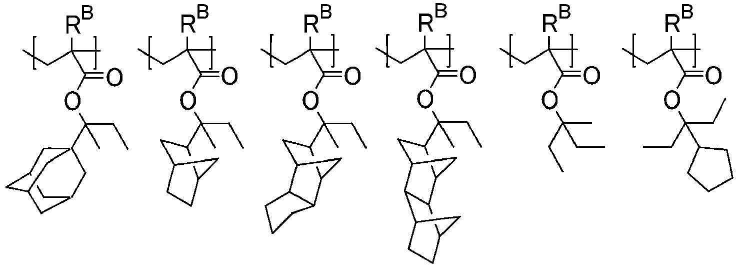

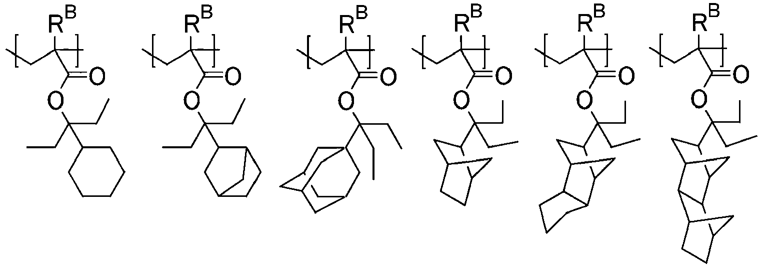

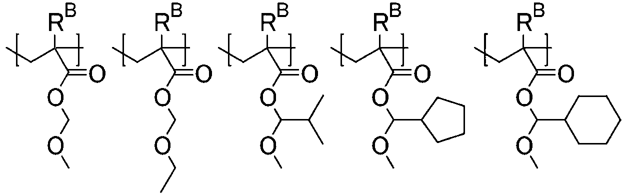

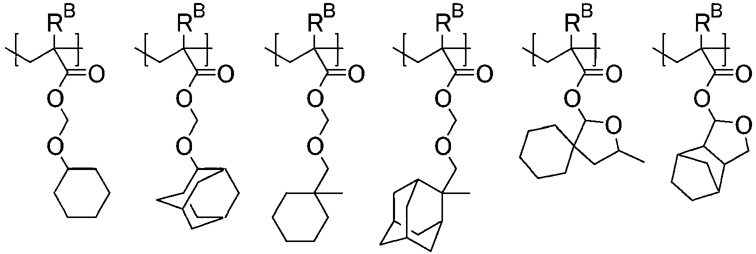

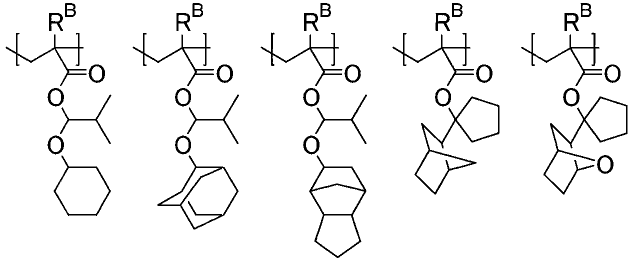

It should be noted that the above specific examples apply to the case where Z^{B} is a single bond, but the same acid-labile groups can also be combined with Z^{B} other than a single bond.

Examples of the repeating unit represented by formula (b) include those shown below, but are not limited thereto. It should be noted that, in the following formulae, R^{B} is a hydrogen atom, a fluorine atom, a methyl group, or a trifluoromethyl group.

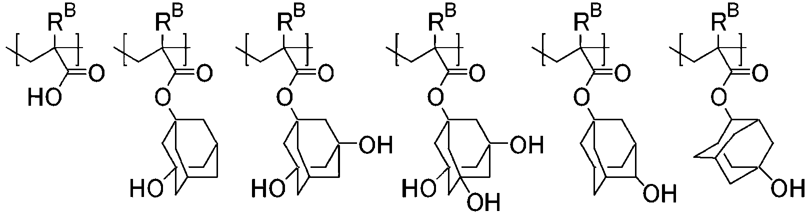

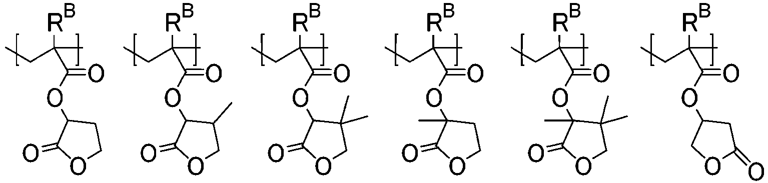

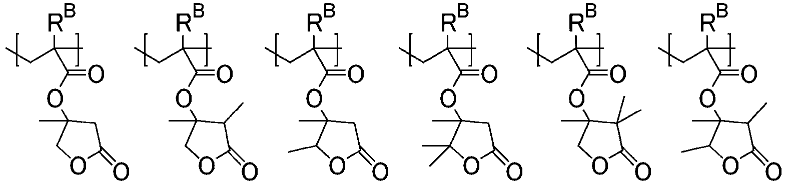

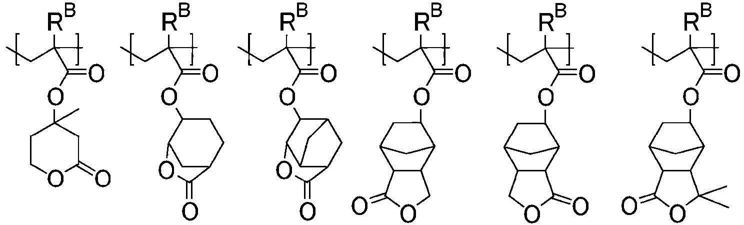

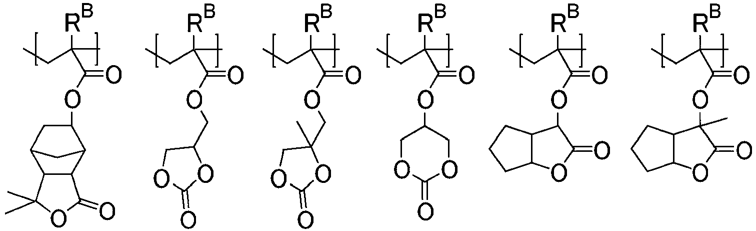

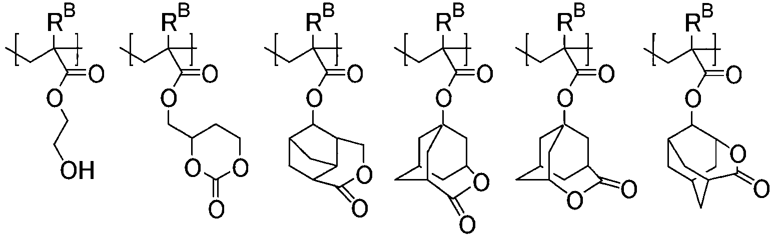

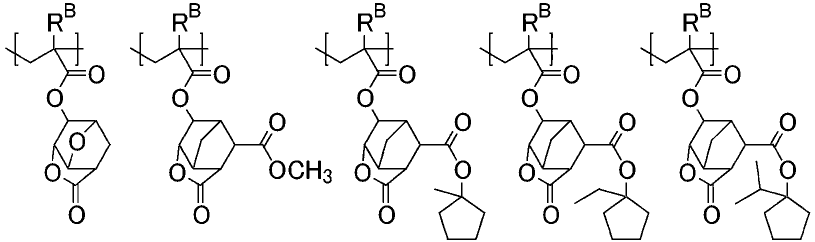

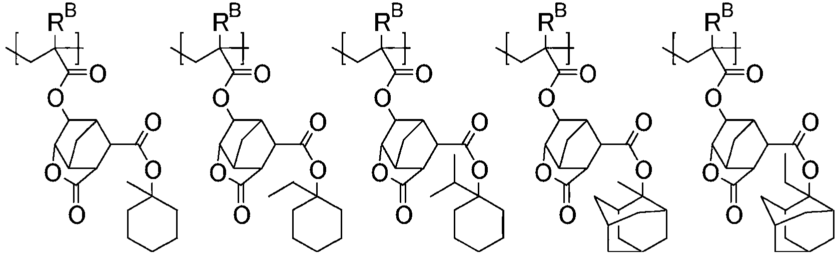

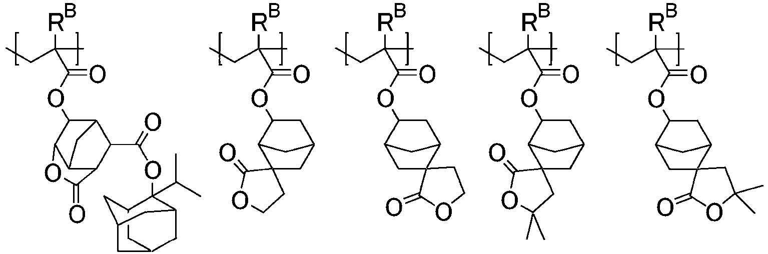

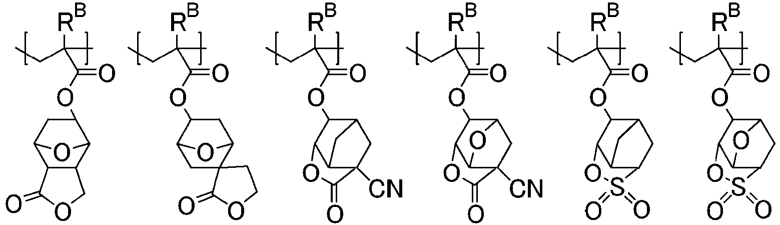

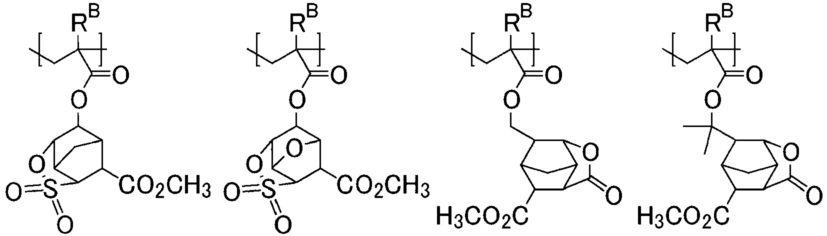

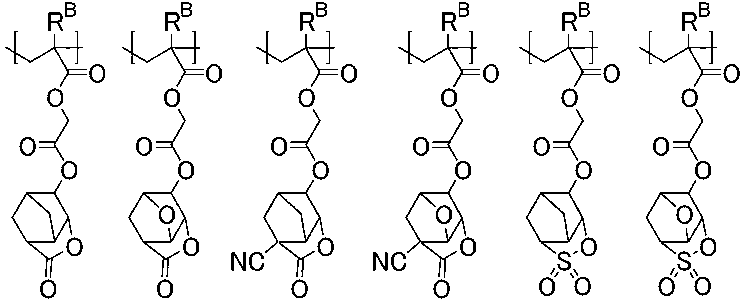

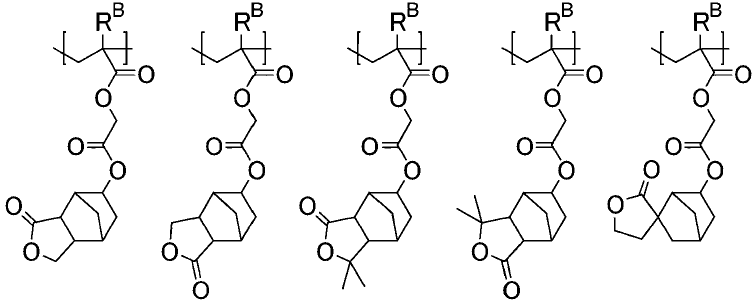

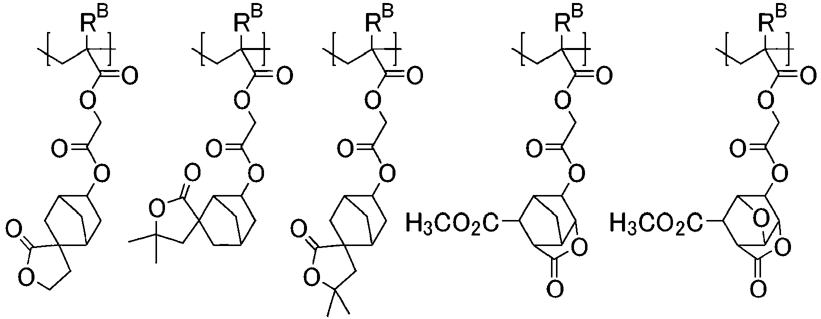

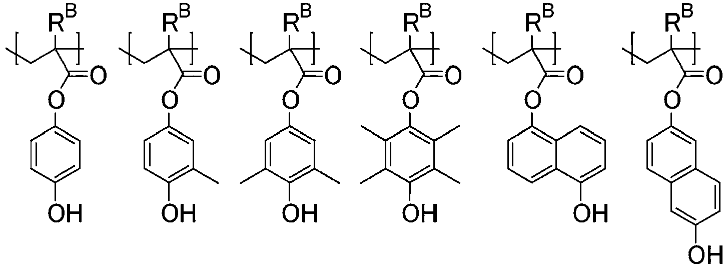

The base resin in the composition for forming a resist pattern of the present disclosure may contain one or two or more of the above structures.

In the case where the base resin contains two or more of the above structures, R^{B}, X^{B}, Y^{B}, Z^{B}, and R^{X} in each structure may be the same or different.

The weak-acid PAG used may be a photodegradable base that can be exposed to generate a weak acid. Examples of the photodegradable base include onium salt compounds that can be decomposed by exposure. Examples of the onium salt compounds include a sulfonium salt compound represented by the following formula (10-1) and an iodonium salt compound represented by the following formula (10-2): 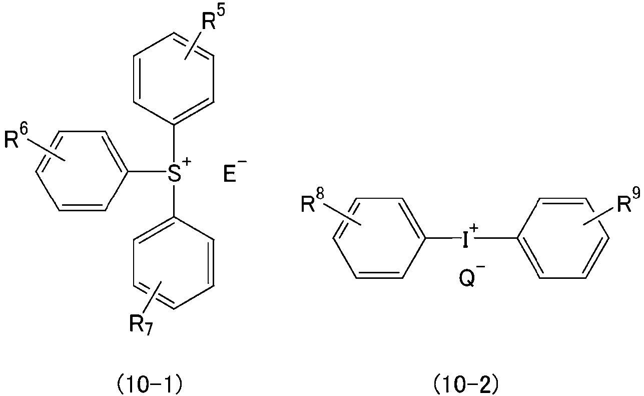
wherein, in formulae (10-1) and (10-2), R⁵ to R⁹ are each independently a hydrogen atom, an alkyl group, an alkoxy group, a hydroxy group, or a halogen atom; and
E⁻ and Q⁻ are each independently OH⁻, R^{β}-COO⁻, R^{β}-SO₃⁻, or an anion represented by the following formula (10-3), provided that R^{β} is an alkyl group, an aryl group, or an aralkyl group, 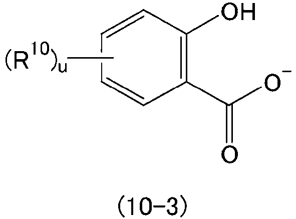
wherein, in formula (10-3), R¹⁰ is a C1-C12 linear or branched alkyl group in which some or all of the hydrogen atoms are optionally replaced by fluorine atoms, or a C1-C12 linear or branched alkoxyl group; and u is an integer of 0 to 2.

Specific examples of the weak-acid PAG include the following compounds.

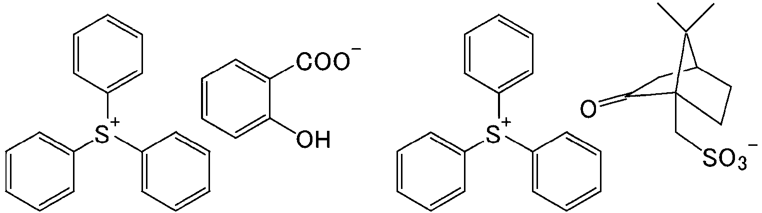

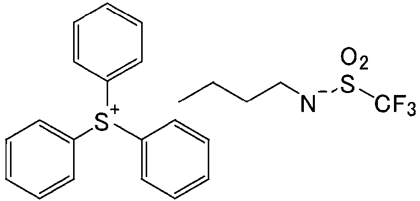

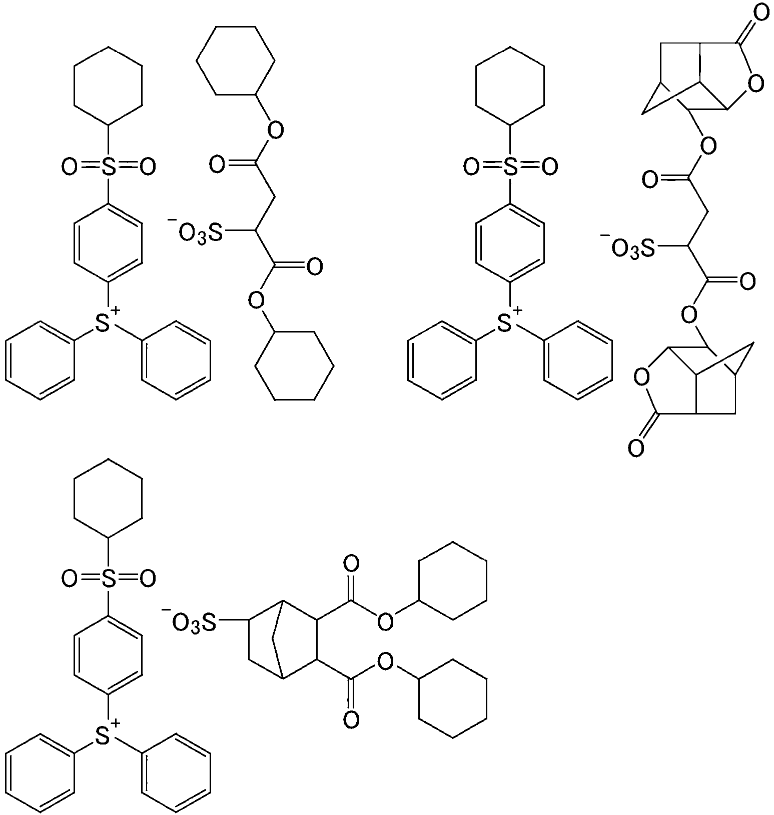

The resist pattern can be obtained through: a step of providing the composition for forming a resist pattern; a film formation step including applying the composition onto a substrate to form a film; an exposure step including exposing the film to electromagnetic waves or high-energy rays having a wavelength of 300 nm or less through a photomask to transfer the pattern of the photomask to the film; and a development step including developing the film using a developer to obtain the pattern.

### <<Composition for Forming Resist Upper Layer Film>>

As described above, the composition for forming a resist upper layer film of the present disclosure preferably contains the fluorine-containing polymer of the present disclosure and a solvent. Examples of the solvent include those that are the same as the preferable solvents described for the composition for forming a resist pattern of the present disclosure.

An exemplary method for forming a resist pattern using such a composition for forming a resist upper layer film may include: a step of providing the composition for forming a resist upper layer film; a film formation step including applying the composition for forming a resist upper layer film onto a resist film to form a resist upper layer film; an exposure step including exposing the resist upper layer film and the resist film to electromagnetic waves or high-energy rays having a wavelength of 300 nm or less through a photomask to transfer the pattern of the photomask to the resist film; and a development step including removing the resist upper layer film and developing the resist film using a developer to obtain the pattern.

### <Formation of Material (Waste Material) That Was Resist Film or Upper Layer Film>

After baking the exposed substrate, a development process is performed using a developer. Development refers to the formation of a resist pattern using an alkaline solution (for positive patterning) or an organic solvent (for negative patterning) as the developer.

### [Positive Patterning]

Positive patterning refers to the formation of a pattern using an alkaline solution as the developer to dissolve and wash away only the exposed portion.

Usually, an aqueous solution of tetramethylammonium hydroxide with a concentration that is 0.1% by mass or more and 10% by mass or less can be used as the alkaline aqueous solution for the developer.

Examples of other alkaline developers include alkaline aqueous solutions in which any of the following is dissolved: sodium hydroxide, potassium hydroxide, sodium carbonate, sodium silicate, sodium metasilicate, ammonia, ethylamine, n-propylamine, diethylamine, di-n-propylamine, triethylamine, methyldiethylamine, dimethylethanolamine, triethanolamine, tetramethylammonium hydroxide, tetraethylammonium hydroxide, pyrrole, piperidine, choline, 1,8-diazabicyclo-[5,4,0]-7-undecene, 1,5-diazabicyclo-[4,3,0]-5-nonane, and the like.

The developer to be used is not limited as long as it can remove the desired resist or resist upper layer film by a predetermined developing method, and typical examples include alkali aqueous solutions using inorganic alkalis, primary, secondary, or tertiary amines, quaternary ammonium salts, and mixtures thereof. In addition, water-soluble organic solvents, such as alcohols, including methanol and ethanol, and surfactants can also be added in appropriate amounts to these developers.

In the case where development is carried out using such an alkaline aqueous solution, washing, rinsing, drying, and other processes, if necessary, can be performed to form the desired pattern.

As the developing method, a known method such as immersion method, paddle method, or spray method can be used, and the development time may be 0.1 minutes or longer and 3 minutes or shorter. Also, the development time is preferably 0.5 minutes or longer and 2 minutes or shorter. During the development, the upper layer film may be dissolved, and only the exposed portion of the underlying resist film may be dissolved.

The fluorine-containing polymer may be removed from the substrate in the alkali development step or resist removal step. The material removed from the substrate, which was the resist film or upper layer film, is a waste material. The waste material contains the fluorine-containing polymer of the present disclosure.

### [Negative Patterning]

Negative patterning refers to the formation of a pattern using an organic solvent as the developer to dissolve and wash away only the unexposed portion.

Usually, butyl acetate can be used as the organic solvent for the developer.

Examples of other organic solvents include 2-octanone, 2-nonanone, 2-heptanone, 3-heptanone, 4-heptanone, 2-hexanone, 3-hexanone, diisobutyl ketone, methylcyclohexanone, acetophenone, methylacetophenone, propyl acetate, butyl acetate, isobutyl acetate, pentyl acetate, butenyl acetate, isopentyl acetate, propyl formate, butyl formate, isobutyl formate, pentyl formate, isopentyl formate, methyl valerate, methyl pentenoate, methyl crotonate, ethyl crotonate, methyl propionate, ethyl propionate, ethyl 3-ethoxypropionate, methyl lactate, ethyl lactate, propyl lactate, butyl lactate, isobutyl lactate, pentyl lactate, isopentyl lactate, methyl 2-hydroxyisobutyrate, ethyl 2-hydroxyisobutyrate, methyl benzoate, ethyl benzoate, phenyl acetate, benzyl acetate, methyl phenylacetate, ethyl phenylacetate, benzyl formate, phenylethyl formate, methyl 3-phenylpropionate, benzyl propionate, and 2-phenylethyl acetate.

The developer to be used is not limited as long as it can remove the desired resist or resist upper layer film by a predetermined developing method. Water-soluble organic solvents, such as alcohols, including methanol and ethanol, and surfactants can also be added in appropriate amounts to such developers.

In the case where development is carried out using such an organic solvent, washing, rinsing, drying, and other processes, if necessary, can be performed to form the desired negative pattern.

As the developing method, a known method such as immersion method, paddle method, or spray method can be used, and the development time may be 0.1 minutes or longer and 3 minutes or shorter. Also, the development time is preferably 0.5 minutes or longer and 2 minutes or shorter. During the development, the upper layer film may be dissolved, and only the exposed portion of the underlying resist film may be dissolved.

The fluorine-containing polymer may be removed from the substrate in the development step or resist removal step. The material removed from the substrate, which was the resist film or upper layer film, is a waste material. The waste material contains the fluorine-containing polymer of the present disclosure.

The waste liquid generated in the development step or the subsequent rinsing step, which contains the fluorine-containing polymer of the present disclosure, may be disposed of by heating through complete combustion. Alternatively, it may be disposed of by heating during the distillation for recovering water from the waste liquid.

Such heating can decompose the monochloro-difluoro-alkyl groups in the fluorine-containing polymer, resulting in the decomposition of the fluorine-containing polymer. It should be noted that, since it has been confirmed that fluoride ions and chloride ions are generated by the heating process, it is estimated that the monochloro-difluoro-alkyl groups have been decomposed to convert to carboxy groups.

The temperature of such heating is preferably 50°C to 200°C, more preferably 55°C to 160°C, still more preferably 60°C to 120°C.

The temperature on the upper limit side may be set lower, for example, at 100°C or lower, or even 95°C or lower.

Examples of methods for disposal at such a low heating temperature include precipitation, adsorption, and activated sludge methods. The above methods are simpler than combustion, and it is easy to dispose of the waste material derived from the fluorine-containing polymer of the present disclosure.

Therefore, by using the fluorine-containing polymer of the present disclosure, it is possible to contribute to energy reduction in semiconductor processes.

### EXAMPLES

The present invention will be described in detail with reference to examples below. However, the present invention is not limited to the following examples.

In the examples, unless otherwise noted, some compounds are denoted as follows.

Methyl ethyl ketone: MEK
Diisobutyl ketone: IPE
Tetrahydrofuran: THF
Tetramethylammonium hydroxide: TMAH
Propylene glycol monomethyl ether acetate: PGMEA
MA-4FHB-OH: compound represented by the following chemical formula: 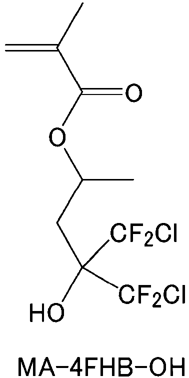
4FIP-M: compound represented by the following chemical formula: 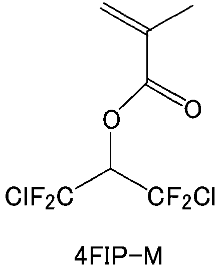
i-4FHK-OH: compound represented by the following chemical formula: 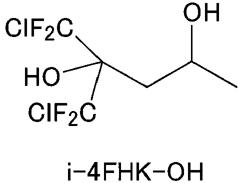
4FIP: compound represented by the following chemical formula: 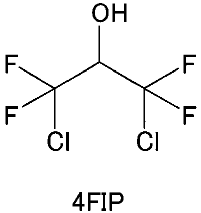
MA-BTHB-OH: compound represented by the following chemical formula: 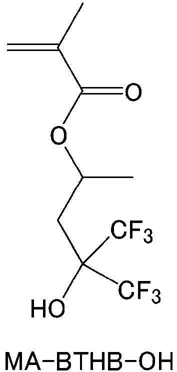
HFIP-M: compound represented by the following chemical formula: 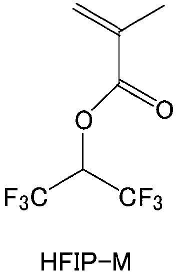
4FHB-NB: compound represented by the following chemical formula: 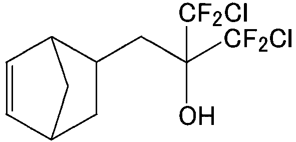
MA-4FHB-NB: compound represented by the following chemical formula: 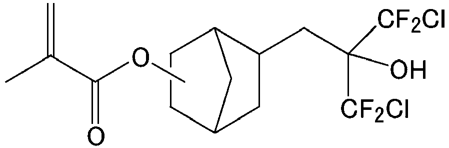
MA-4FHP-OH: compound represented by the following chemical formula: 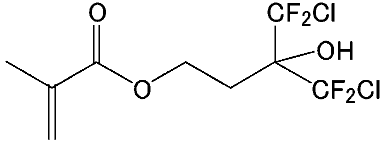
4F-Iee: compound represented by the following chemical formula: 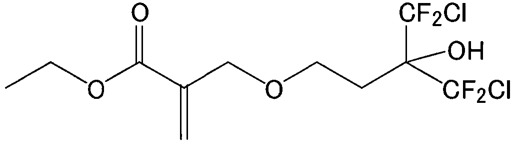
MA-MIP-4FA: compound represented by the following chemical formula: 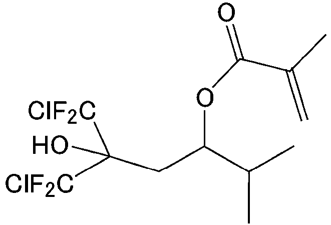
Bis-4FIP form: compound represented by the following chemical formula:

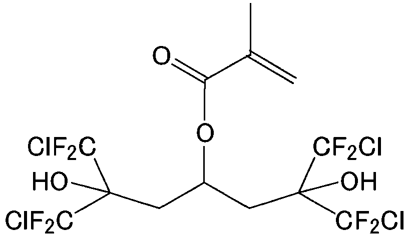

The apparatuses and measurement conditions used for various analyses will be described.

### [Analysis of Fluorine-Containing Polymerizable Monomer]

¹⁹F-NMR and ¹H-NMR measurements were performed using a nuclear magnetic resonance apparatus with a resonance frequency of 400 MHz (hereinafter referred to as NMR, manufactured by JEOL Ltd., equipment name: JNM-ECA400).

### [Analysis of Polymer]

The composition of repeating components in the polymers were determined from the ¹H-NMR and ¹⁹F-NMR measurements obtained by NMR.

### [Analysis of Weight Average Molecular Weight]

The weight average molecular weight (Mw) of the resin compositions and the like described below was measured as follows. The number average molecular weight Mn and molecular weight distribution (the ratio of the mass average molecular weight Mw to the number average molecular weight Mn = Mw/Mn) of the polymers were measured using high performance gel permeation chromatography (hereinafter also referred to as GPC; manufactured by Tosoh Corporation, model HLC-8320GPC) with one ALPHA-M column and one ALPHA-2500 column (both manufactured by Tosoh Corporation) connected in series, and tetrahydrofuran used as the eluent. A differential refractive index detector was used as the detector.

The synthesis of fluorine-containing polymerizable monomers and intermediates for the fluorine-containing polymerizable monomers will be described.

### [Synthesis Example 1: Synthesis of i-4FHK-OH and MA-4FHB-OH]

To a 1 L three-neck eggplant flask were added concentrated sulfuric acid (0.31 g, 3.16 mmol), acetone (307 g, 5.28 mol), and 1,3-dichlorotetrafluoroacetone (manufactured by SynQuest Laboratories Inc., 210 g, 1.06 mol), and the mixture was stirred at 30°C overnight. The reaction solution was concentrated with an evaporator at a bath temperature of 30°C under 100 hPa, and the resulting concentrate was washed by adding ion-exchanged water. Thereafter, concentration was performed with an evaporator to obtain a concentrate 1 (245 g).

To a 1 L three-neck eggplant flask were added tetrahydrofuran (super dehydrated grade, 551 g) and sodium borohydride (32.5 g, 857 mmol), and they were dispersed and then cooled in an ice bath. Thereafter, the concentrate 1 was added dropwise over 30 minutes, and after 1.5 hours, the reaction was terminated with aqueous hydrochloric acid. The reaction solution was extracted with IPE (551 g) and washed once with ion-exchanged water. The washed IPE solution was concentrated with an evaporator at a bath temperature of 42°C under 20 hPa while replacing the solvent with toluene, yielding a concentrate 2 (215 g) containing i-4FHK-OH.

To a 1 L three-neck eggplant flask were added the concentrate 2, methanesulfonic acid (5.97 g, 62.1 mmol), and NONFLEX MBP (manufactured by Seiko Chemical Co., Ltd., 0.96 g), and they were mixed, followed by dropwise addition of methacrylic anhydride (hereinafter referred to as MAAH, reagent from Tokyo Chemical Industry Co., Ltd., 105 g, 683 mmol) at a bath temperature of 38°C. Thereafter, the mixture was stirred at a bath temperature of 47°C for 1.5 hours and at a bath temperature of 57°C for 3 hours, and the reaction was terminated. The reaction solution was extracted with IPE (322 g), and washed once each with aqueous sodium hydroxide and ion-exchanged water. Thereafter, a small amount of 2-methoxyphenothiazine was added, and concentration was performed with an evaporator at a bath temperature of 42°C under 20 hPa. The resulting concentrate was distilled under 0.2 kPa at an internal temperature of 125°C to 136°C to obtain MA-4FHB-OH (123 g, yield 36% (yield over three steps), GC purity >99%). The present reaction is shown below.

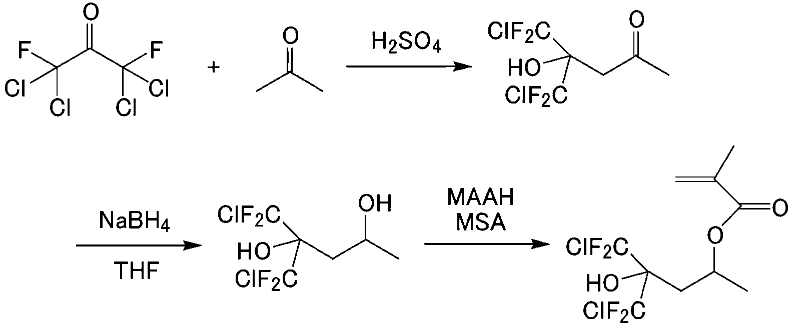

### <NMR Analysis Results>

The results of nuclear magnetic resonance analysis (hereinafter sometimes referred to as NMR) are shown below.
¹H-NMR (CDCl₃, Reference substance: TMS): 6.36 ppm (s, 1H), 6.18 ppm (m, 1H), 5.65 ppm (quin, J = 1.6 Hz, 1H), 5.22 ppm (m, 1H), 2.43 ppm (dd, J = 4.8 Hz, 0.8 Hz, 2H), 1.95 ppm (m, 3H), 1.46 ppm (d, J = 6.4 Hz, 3H)
¹⁹F-NMR (CDCl₃, Reference substance: C₆F₆): -60.1 ppm (q, 15.2 Hz, 2F), -79.6 ppm (m, 2F)

### [Synthesis Example 2: Synthesis of 4FIP and 4FIP-M]

To a 1 L three-neck eggplant flask were added sodium borohydride (22.8 g, 603 mmol) and IPE (300 g), and the mixture was cooled in an ice bath. Thereafter, 1,3-dichlorotetrafluoroacetone (manufactured by SynQuest Laboratories Inc., 100 g, 503 mmol) was slowly added dropwise, and the mixture was then stirred at room temperature for 17 hours. After cooling in an ice bath, the reaction was terminated with 200 g of aqueous hydrochloric acid. Thereafter, the organic layer obtained by liquid-liquid separation was subjected to distillation under normal pressure (oil bath 120°C to 150°C), thereby obtaining a solution of 4FIP in IPE (129 g). The purity was 64 wt%, and the yield was 82%. The present reaction is shown below.

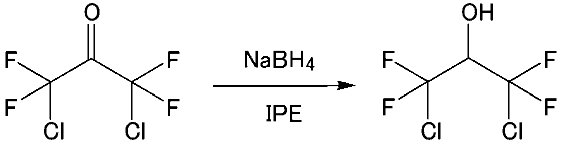

### <NMR Analysis Results>

The results of NMR are shown below. The results for 4FIP only are shown. ¹H-NMR (CDCl₃, Reference substance: TMS): 4.42 ppm (m, 1H) ¹⁹F-NMR (CDCl₃, Reference substance: C₆F₆): -58.7 ppm (m, 2F), -63.4 ppm (m, 2F)

To a 500 mL three-neck eggplant flask were added the solution of 4FIP in IPE (120 g, 410 mmol), triethylamine (46 g, 454 mmol), and IPE (150 g), and the mixture was cooled in an ice bath. Thereafter, methacryloyl chloride (45 g, 45.2 mmol) was slowly added dropwise, and the mixture was then stirred at room temperature for 3 hours, after which the reaction was terminated with 200 g of saturated aqueous sodium bicarbonate. Thereafter, the organic layer obtained by liquid-liquid separation was washed once with 200 g of saturated aqueous sodium bicarbonate and twice with 200 g of distilled water, and then subjected to distillation under reduced pressure (pressure reduction 0.6 kPa, oil bath 50°C to 100°C) to obtain 86 g of 4FIP-M. The yield was 80%, and the GC purity was >99%. The present reaction is shown below.

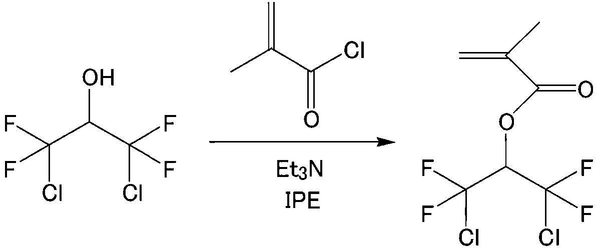

### <NMR Analysis Results>

The results of NMR are shown below.
¹H-NMR (CDCl₃, Reference substance: TMS): 6.36 ppm (m, 1H), 5.89 ppm (m, 1H), 5.84 ppm (m, 1H), 2.03 ppm (m, 3H)
¹⁹F-NMR (CDCl₃, Reference substance: C₆F₆): -58.5 ppm (m, 2F), -60.1 ppm (m, 2F)

### [Synthesis Example 3: Synthesis of 4FHB, 4FHB-NB, and MA-4FHB-NB]

A pressure-resistant vessel (500 mL) equipped with a stirrer was charged with 1,3-dichlorotetrafluoroacetone (manufactured by SynQuest Laboratories Inc., 50 g, 0.25 mol), and the reactor was sealed. Next, the interior of the reactor was degassed with a vacuum pump, and propylene (10.5 g, 0.25 mol) was introduced while stirring the contents with the stirrer. The temperature inside the reactor was then raised to 100°C, and stirring was continued for 80 hours. After completion of the reaction, distillation under reduced pressure was performed at an absolute pressure of 3.0 kPa and a temperature of 68°C to 69°C to obtain 1-chloro-2-(chlorodifluoromethyl)-1,1-difluoro-3-buten-1-ol (hereinafter referred to as 4FHB) represented by the following formula (36.5 g, yield 60%). The present reaction is shown below.

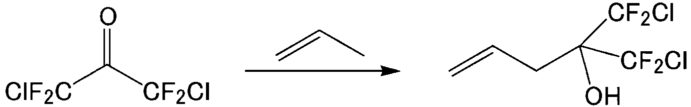

### <NMR Analysis Results>

The results of nuclear magnetic resonance analysis (hereinafter sometimes referred to as NMR) are shown below.
¹H-NMR (CDCl₃, Reference substance: TMS) δ 5.89 (m, 1H), 5.40 (ddt, 1H), 5.34 (dq, 1H), 3.20 (brs, 1H), 2.88 (dd, 2H)
¹⁹F-NMR (CDCl₃, Reference substance: C₆F₆): δ -59.7 (s, 4F)

A 100 mL SUS autoclave was charged with dicyclopentadiene (13.22 g, 0.1 mol) and 4FHB (24.3 g, 0.1 mol). The reaction mixture was stirred for 1 hour and heated at 150°C for 10 hours. After cooling the reaction mixture, the components were fractionally distilled. The fraction distilled at a pressure of 0.1 kPa and a temperature of 85°C to 90°C was collected to obtain 15 g of 4FHB-NB shown below, in a yield of 40%. The present reaction is shown below.

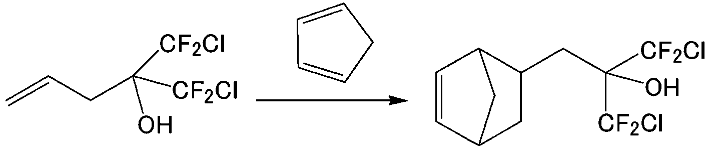

### <NMR Analysis Results>

The results of nuclear magnetic resonance analysis (hereinafter sometimes referred to as NMR) are shown below.
¹H-NMR (CDCl₃, Reference substance: TMS) δ 6.20 (dd, 1H), 5.98 (dd, 1H), 3.05 (m, 1H), 2.92 (m, 1H), 2.81 (m, 1H), 2.00 (dd, 2H), 1.80 (d, 1H), 1.35 (m, 2H)
¹⁹F-NMR (CDCl₃, Reference substance: C₆F₆): δ -59.9 (m, 2F), -60.5 (m, 2F)

To a 100 mL three-neck eggplant flask were added 4FHB-NB (10 g, 31.7 mmol) and formic acid (30 g, 653 mmol), and the mixture was stirred at 65°C for 2 hours. Thereafter, IPE (100 g) was added, and the organic layer was washed once with 100 g of ion-exchanged water, twice with 100 g of saturated aqueous sodium bicarbonate, and once with 100 g of ion-exchanged water. Then, the organic layer obtained by liquid-liquid separation was concentrated with an evaporator to obtain a concentrate 3 (10.5 g). The present reaction is shown below.

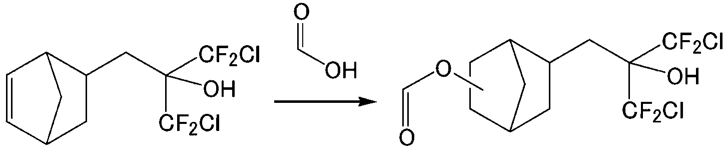

To a 300 mL three-neck eggplant flask were added the concentrate 3 (10.5 g), methanol (30 g), and ion-exchanged water (30 g), and then trifluoroacetic acid (0.3 g, 2.6 mmol) was added, followed by stirring at 60°C for 8 hours. Thereafter, IPE (100 g) was added, and the organic layer was washed once with 100 g of ion-exchanged water, once with 100 g of saturated aqueous sodium bicarbonate, and once with 100 g of ion-exchanged water. Then, the organic layer obtained by liquid-liquid separation was concentrated with an evaporator to obtain a concentrate 4 (9.5 g). The present reaction is shown below.

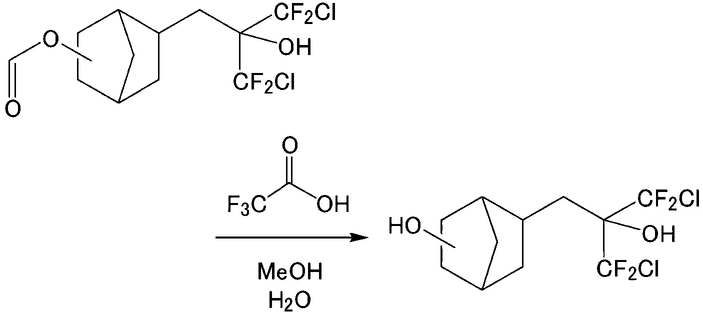

To a 100 mL three-neck eggplant flask were added the concentrate 4 (9.5 g), toluene (20 g), and methacrylic anhydride (4.7 g, 30.8 mmol), and then methanesulfonic acid (0.4 g, 4.4 mmol) was added, followed by stirring at 80°C for 1 hour. Thereafter, IPE (20 g) was added, and the organic layer was washed once with 50 g of saturated aqueous sodium bicarbonate and once with 50 g of ion-exchanged water. Then, the organic layer obtained by liquid-liquid separation was concentrated with an evaporator. Thereafter, Kugelrohr distillation was performed to obtain 5.1 g of MA-4FHB-NB. The purity was 98%, and the yield from 4FHB-NB was 50%. The present reaction is shown below.

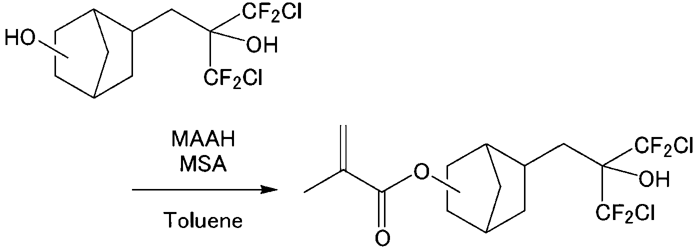

### <NMR Analysis Results>

The results of nuclear magnetic resonance analysis (hereinafter sometimes referred to as NMR) are shown below.
¹H-NMR (CDCl₃, Reference substance: TMS) δ 6.05 (m, 1H), 5.52 (m, 1H),
4.64 (m, 1H), 3.55 (m, 1H), 2.33 (m, 2H), 2.15 (m, 2H),
1.95 (m, 1H), 1.90 (m, 3H), 1.88 (m, 1H), 1.53 (m, 2H),
1.30 (m, 2H), 1.15 (m, 1H)
¹⁹F-NMR (CDCl₃, Reference substance: C₆F₆): δ -60.3 (m, 4F)

### [Synthesis Example 4: Synthesis of 4FHP-OH and MA-4FHP-OH]

To a 300 mL three-neck eggplant flask were added 1,3-dichlorotetrafluoroacetone (manufactured by SynQuest Laboratories Inc., 50 g, 252 mmol) and butyl vinyl ether (40.2 g, 400 mmol), and the mixture was stirred at room temperature for 18 hours. Thereafter, simple distillation was carried out to obtain 50.9 g of the target oxetane with a GC purity of 95% and a yield of 83%. The present reaction is shown below.

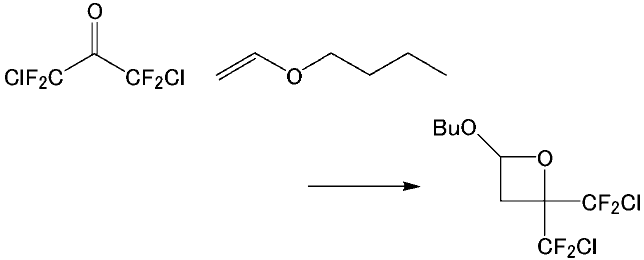

### <NMR Analysis Results>

The results of nuclear magnetic resonance analysis (hereinafter sometimes referred to as NMR) are shown below.
¹H-NMR (CDCl₃, Reference substance: TMS) δ 5.56 (t, 1H), 3.79 (dt, 1H), 3.55 (dt, 1H), 3.11 (dd, 1H), 2.93 (dd, 1H), 1.60 (m, 2H), 1.40 (m, 2H), 0.93 (t, 3H)
¹⁹F-NMR (CDCl₃, Reference substance: C₆F₆): δ -62.2 (m, 4F)

To a 300 mL three-neck eggplant flask were added the oxetane (49 g, 252 mmol), ion-exchanged water (72 g), and formic acid (180 g), and the mixture was stirred at 70°C for 4 hours. Thereafter, IPE (200 g) was added, liquid-liquid separation was performed, and the resulting organic layer was subjected to concentration with an evaporator and simple distillation to finally obtain 34 g of the target aldehyde with a GC purity of 98% and a yield of 54%. The present reaction is shown below.

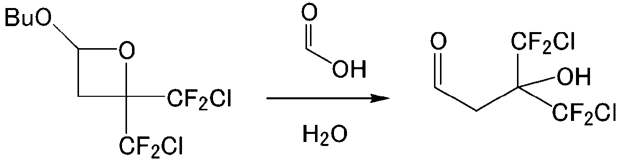

### <NMR Analysis Results>

The results of nuclear magnetic resonance analysis (hereinafter sometimes referred to as NMR) are shown below.
¹H-NMR (CDCl₃, Reference substance: TMS) δ 9.88 (s, 1H), 3.12 (s, 2H)
¹⁹F-NMR (CDCl₃, Reference substance: C₆F₆): δ -60.8 (d, 2F), -62.6 (d, 2F)

To a 300 mL three-neck eggplant flask were added the aldehyde (33 g, 252 mmol), IPE (100 g), and sodium borohydride (51 g, 135 mmol), and the mixture was stirred at room temperature for 7 hours. Thereafter, IPE (200 g) and 1N aqueous hydrochloric acid solution (200 g) were added, liquid-liquid separation was performed, and the organic layer was concentrated with an evaporator and distilled to obtain 30 g of 4FHP-OH with a GC purity of 99% and a yield of 90%. The present reaction is shown below.

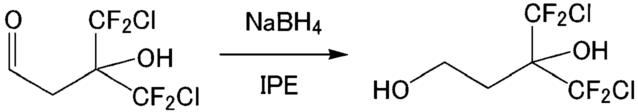

### <NMR Analysis Results>

The results of nuclear magnetic resonance analysis (hereinafter sometimes referred to as NMR) are shown below.
¹H-NMR (CDCl₃, Reference substance: TMS) δ 6.10 (brs, 1H), 4.15 (t, 2H), 2.43 (brs, 1H), 2.38 (t, 2H)
¹⁹F-NMR (CDCl₃, Reference substance: C₆F₆): δ -61.1 (s, 2F), -61.5 (s, 2F)

To a 50 mL three-neck eggplant flask were added 4FHP-OH (4.2 g, 17.1 mmol), toluene (15 g), methacrylic anhydride (2.8 g, 18.0 mmol), and methanesulfonic acid (0.4 g, 4.4 mmol), and the mixture was stirred at 70°C for 7 hours. Thereafter, IPE (20 g) was added, the mixture was washed with saturated aqueous sodium bicarbonate and with ion-exchanged water, and the resulting organic layer was subjected to concentration with an evaporator and simple distillation to obtain 3.1 g of MA-4FHP-OH with a GC purity of 99% and a yield of 69%. The present reaction is shown below.

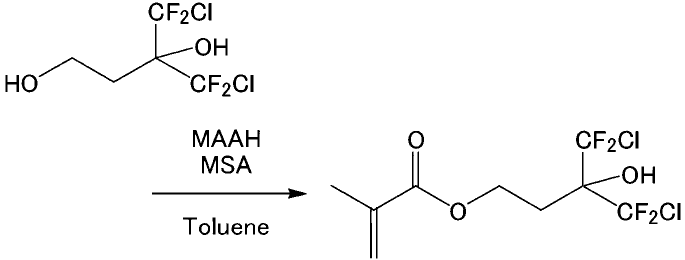

### <NMR Analysis Results>

The results of nuclear magnetic resonance analysis (hereinafter sometimes referred to as NMR) are shown below.
¹H-NMR (CDCl₃, Reference substance: TMS) δ 6.14 (m, 1H), 5.63 (m, 1H), 4.88 (brs, 1H), 4.46 (t, 2H), 2.48 (t, 2H), 1.93 (m, 3H)
¹⁹F-NMR (CDCl): δ -61.0 (m, 4F)

### [Synthesis Example 5: Synthesis of 4F-Iee]

To a 50 mL three-neck eggplant flask were added 4FHP-OH (8.1 g, 33 mmol), dichloromethane (30 g), and triethylamine (4.0 g, 39.3 mmol), and the mixture was cooled in an ice bath. Thereafter, ethyl 2-bromomethylacrylate (6.4 g, 33 mmol) was added, and the mixture was stirred at room temperature for 3 hours. The reaction solution was filtered under reduced pressure, and the filtrate was washed with 1N aqueous hydrochloric acid solution (50 g), ion-exchanged water (50 g), and saturated saline. Then, the resulting organic layer was subjected to concentration with an evaporator and simple distillation to obtain 12.8 g of 4F-Iee with a GC purity of 99% and a yield of 80%. The present reaction is shown below.

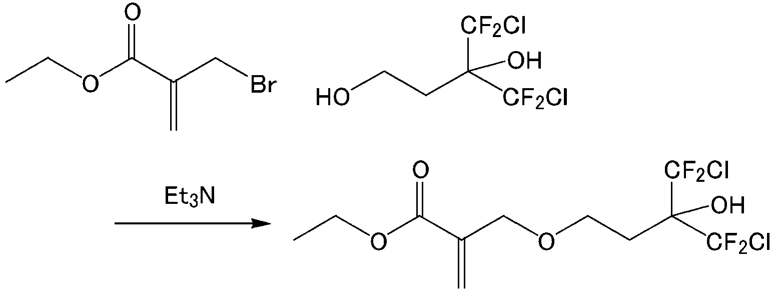

### <NMR Analysis Results>

The results of nuclear magnetic resonance analysis (hereinafter sometimes referred to as NMR) are shown below.
¹H-NMR (CDCl₃, Reference substance: TMS) δ 6.35 (m, 1H), 5.88 (brs, 1H), 5.81 (m, 1H), 4.23 (q, 2H), 4.22 (brs, 2H), 3.91 (t, 2H), 2.41 (t, 2H), 1.30 (t, 3H)
¹⁹F-NMR (CDCl₃, Reference substance: C₆F₆): δ -61.0 (d, 4F)

### [Synthesis Example 6: Synthesis of MA-MIP-4FA]

MA-MIP-4FA was synthesized by the same method as in Synthesis Example 1, except that acetone was changed to isopropyl methyl ketone. The amount obtained was 110 g, with a yield of 30% (yield over three steps) and a GC purity of >99%. The present reaction is shown below.

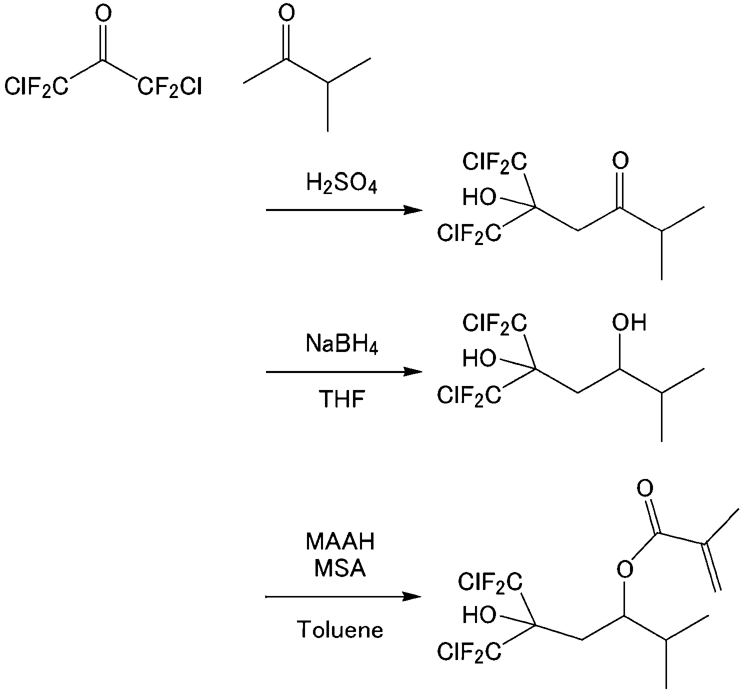

### <NMR Analysis Results>

The results of nuclear magnetic resonance analysis (hereinafter sometimes referred to as NMR) are shown below.
¹H-NMR (CDCl₃, Reference substance: TMS): 6.39 ppm (s, 1H), 6.19 ppm (m, 1H), 5.70 ppm (dt, 1H), 5.20 ppm (m, 1H), 2.45 ppm (dd, 2H), 2.04 (m, 1H), 1.93 ppm (m, 3H), 1.24 ppm (d, 6H)
¹⁹F-NMR (CDCl₃, Reference substance: C₆F₆): -60.0 ppm (q, 2F), -79.6 ppm (m, 2F)

### [Synthesis Example 7: Synthesis of Bis-4FIP Form]

To a 100 mL autoclave were added trifluoroacetic acid (0.14 g, 1.2 mmol), acetone (1.5 g, 25.1 mol), and 1,3-dichlorotetrafluoroacetone (manufactured by SynQuest Laboratories Inc., 14.0 g, 70.4 mol), and the mixture was stirred at 80°C overnight. The reaction solution was concentrated with an evaporator at a bath temperature of 30°C under 100 hPa, and the resulting concentrate was washed by adding ion-exchanged water. Thereafter, concentration with an evaporator was performed to obtain a concentrate 5 (10 g).

To a 100 mL three-neck eggplant flask were added tetrahydrofuran (super dehydrated grade, 30 g) and sodium borohydride (0.9 g, 22 mmol), and they were dispersed, followed by cooling in an ice bath. Thereafter, the concentrate 5 was added dropwise, and after 1.5 hours, the reaction was terminated with aqueous hydrochloric acid. The reaction solution was extracted with IPE (50 g) and washed once with ion-exchanged water. The washed IPE solution was concentrated with an evaporator at a bath temperature of 42°C under 20 hPa while replacing the solvent with toluene, yielding a concentrate 6 (9 g).

To a 100 mL three-neck eggplant flask were added the concentrate 6, methacrylic acid (3.2 g, 37 mmol), concentrated sulfuric acid (0.4 g, 37 mmol), and Topanol A (0.1 g), and the mixture was stirred at 80°C overnight. The reaction solution was extracted with IPE (30 g), and washed once each with saturated aqueous sodium bicarbonate and ion-exchanged water. Thereafter, concentration with an evaporator was performed. The resulting concentrate was distilled to obtain bis-4FIP form (6 g, yield 30% (yield over three steps), GC purity >99%). The present reaction is shown below.

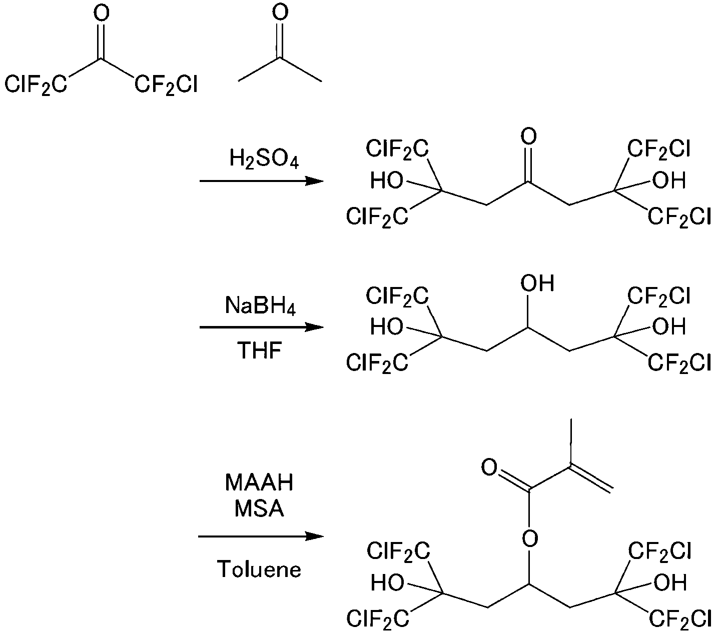

### <NMR Analysis Results>

The results of nuclear magnetic resonance analysis (hereinafter sometimes referred to as NMR) are shown below.
¹H-NMR (CDCl₃, Reference substance: TMS): 6.21 ppm (t, 1H), 5.74 ppm (t, 1H), 5.51 ppm (m, 1H), 4.90 (brt, 1H), 2.55 ppm (m, 4H), 1.96 ppm (t, 3H)
¹⁹F-NMR (CDCl₃, Reference substance: C₆F₆): -61.5 ppm (m, 8F)

Next, the synthesis of polymers will be described.

### [Synthesis Example 8: Synthesis of Fluorine-Containing Polymer 1]

A 100 mL glass flask was charged with 8.0 g (24 mmol) of MA-4FHB-OH and 32 g of MEK at room temperature, followed by the addition of 0.6 g of dimethyl 2,2'-azobis(isobutyrate) (hereinafter referred to as V-601) (manufactured by FUJIFILM Wako Pure Chemical Corporation, 2.4 mmol). Then, the interior of the flask was degassed while stirring. Thereafter, the interior of the flask was replaced with nitrogen gas, the internal temperature was raised to 78°C, and the reaction was performed for 18 hours. The reaction solution was added dropwise to 80 g of heptane for reprecipitation, followed by decantation, and the resulting polymer was dried in a vacuum dryer to obtain 6.4 g of a fluorine-containing polymer 1 having the following repeating unit, with a yield of 80%.

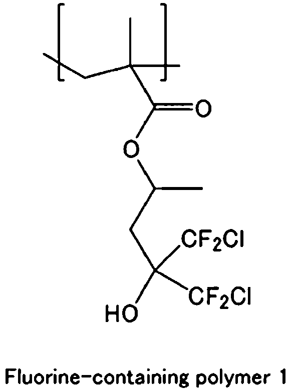

The GPC measurement results were as follows: Mw = 13000 and Mw/Mn = 1.9.

### [Synthesis Example 9: Synthesis of Fluorine-Containing Polymer 2]

A 100 mL glass flask was charged with 5 g (19 mmol) of 4FIP-M and 2.5 g of butyl acetate at room temperature, followed by the addition of 0.2 g (0.9 mmol) of V-601. Then, the interior of the flask was degassed while stirring. Thereafter, the interior of the flask was replaced with nitrogen gas, the internal temperature was raised to 78°C, and the reaction was performed for 18 hours. The reaction solution was added dropwise to 70 g of heptane for reprecipitation, followed by decantation, and the resulting polymer was dried in a vacuum dryer to obtain 4.1 g of a fluorine-containing polymer 2 having the following repeating unit, with a yield of 82%.

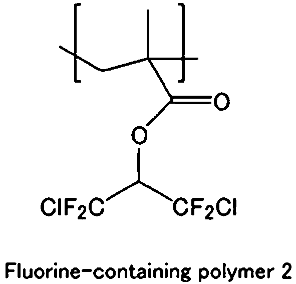

The GPC measurement results were as follows: Mw = 9000 and Mw/Mn = 2.1.

### [Synthesis Example 10: Synthesis of Fluorine-Containing Polymer 3]

A 100 mL glass flask was charged with 8.0 g (24 mmol) of MA-4FHB-OH, 6.3 g (24 mmol) of 4FIP-M, and 60 g of MEK at room temperature, followed by the addition of 1.2 g of V-601 (manufactured by FUJIFILM Wako Pure Chemical Corporation, 4.8 mmol). Then, the interior of the flask was degassed while stirring. Thereafter, the interior of the flask was replaced with nitrogen gas, the internal temperature was raised to 78°C, and the reaction was performed for 18 hours. The reaction solution was added dropwise to 100 g of heptane for reprecipitation, followed by decantation, and the resulting polymer was dried in a vacuum dryer to obtain 12.0 g of a fluorine-containing polymer 3 having the following repeating units, with a yield of 84%.

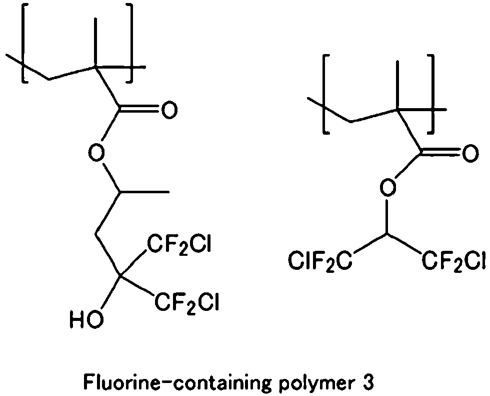

The GPC measurement results were as follows: Mw = 10800 and Mw/Mn = 1.8.

From the NMR measurement results, it was found that the compositional ratio of the structural units in the fluorine-containing polymer 3, expressed in mol%, was the structural unit derived from MA-4FHB-OH:the structural unit derived from 4FIP-M = 52:48.

### [Synthesis Example 11: Synthesis of Fluorine-Containing Polymer 4]

A 100 mL glass flask was charged with 8.0 g (24 mmol) of MA-4FHB-OH, 1.7 g (16 mmol) of vinylsulfonic acid, and 40 g of MEK at room temperature, followed by the addition of 1.2 g of V-601 (manufactured by FUJIFILM Wako Pure Chemical Corporation, 4.8 mmol). Then, the interior of the flask was degassed while stirring. Thereafter, the interior of the flask was replaced with nitrogen gas, the internal temperature was raised to 78°C, and the reaction was performed for 18 hours. The reaction solution was added dropwise to 100 g of heptane for reprecipitation, followed by decantation, and the resulting polymer was dried in a vacuum dryer to obtain 8.0 g of a fluorine-containing polymer 4 having the following repeating units, with a yield of 81%.

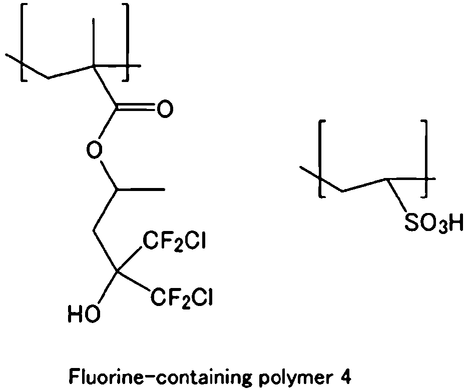

The GPC measurement results were as follows: Mw = 9800 and Mw/Mn = 1.9.

From the NMR measurement results, it was found that the compositional ratio of the structural units in the fluorine-containing polymer 4, expressed in mol%, was the structural unit derived from MA-4FHB-OH:the structural unit derived from vinylsulfonic acid = 59:41.

### [Synthesis Example 12: Synthesis of Fluorine-Containing Polymer 5]

A 100 mL glass flask was charged with 6.3 g (24 mmol) of 4FIP-M, 6.0 g (24 mmol) of 2-ethyl-2-methacryloyloxyadamantane (hereinafter referred to as EMOE), and 50 g of MEK at room temperature, followed by the addition of 1.2 g of V-601 (manufactured by FUJIFILM Wako Pure Chemical Corporation, 4.8 mmol). Then, the interior of the flask was degassed while stirring. Thereafter, the interior of the flask was replaced with nitrogen gas, the internal temperature was raised to 78°C, and the reaction was performed for 18 hours. The reaction solution was added dropwise to 100 g of heptane for reprecipitation, followed by decantation, and the resulting polymer was dried in a vacuum dryer to obtain 10.5 g of a fluorine-containing polymer 5 having the following repeating units, with a yield of 85%.

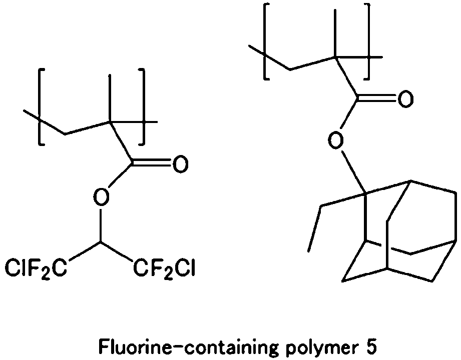

The GPC measurement results were as follows: Mw = 9900 and Mw/Mn = 2.1.

From the NMR measurement results, it was found that the compositional ratio of the structural units in the fluorine-containing polymer 5, expressed in mol%, was the structural unit derived from 4FIP-M:the structural unit derived from EMOA = 55:45.

### [Synthesis Example 13: Synthesis of Fluorine-Containing Polymer 6]

A 200 mL glass flask was charged with 8.0 g (24 mmol) of MA-4FHB-OH, 6.3 g (24 mmol) of 4FIP-M, 6.0 g (24 mmol) of 2-ethyl-2-methacryloyloxyadamantane (hereinafter referred to as EMOE), and 80 g of MEK at room temperature, followed by the addition of 1.2 g of V-601 (manufactured by FUJIFILM Wako Pure Chemical Corporation, 4.8 mmol). Then, the interior of the flask was degassed while stirring. Thereafter, the interior of the flask was replaced with nitrogen gas, the internal temperature was raised to 78°C, and the reaction was performed for 18 hours. The reaction solution was added dropwise to 150 g of heptane for reprecipitation, followed by decantation, and the resulting polymer was dried in a vacuum dryer to obtain 18.1 g of a fluorine-containing polymer 6 having the following repeating units, with a yield of 89%.

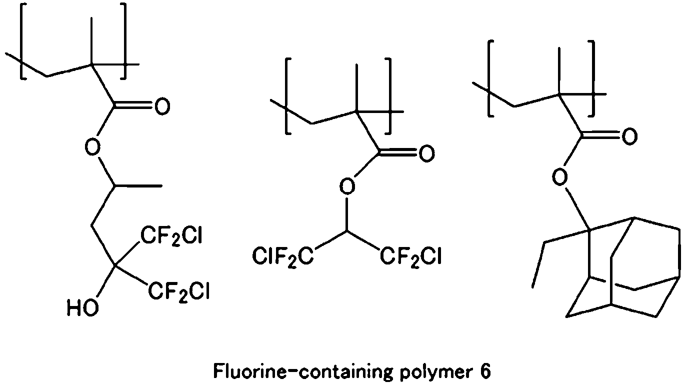

The GPC measurement results were as follows: Mw = 12000 and Mw/Mn = 2.3.

From the NMR measurement results, it was found that the compositional ratio of the structural units in the fluorine-containing polymer 6, expressed in mol%, was the structural unit derived from MA-4FHB-OH:the structural unit derived from 4FIP-M:the structural unit derived from EMOA = 35:33:32.

### [Synthesis Example 14: Synthesis of Fluorine-Containing Polymer 7]

A fluorine-containing polymer was synthesized in the same manner as in Synthesis Example 8, except that MA-4FHB-OH was changed to MA-BTHB-OH. Thus, 6.0 g of a fluorine-containing polymer 7 having the following repeating unit was obtained in a yield of 76%.

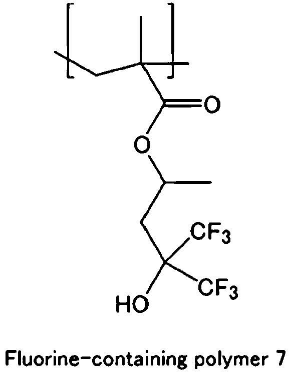

The GPC measurement results were as follows: Mw = 12000 and Mw/Mn = 2.0.

### [Synthesis Example 15: Synthesis of Fluorine-Containing Polymer 8]

A fluorine-containing polymer was synthesized in the same manner as in Synthesis Example 9, except that 4FIP-M was changed to HFIP-M. Thus, 3.9 g of a fluorine-containing polymer 8 having the following repeating unit was obtained in a yield of 80%.

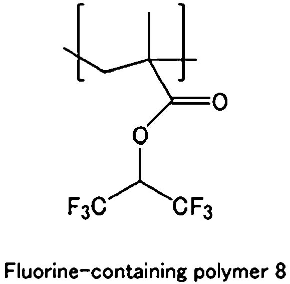

The GPC measurement results were as follows: Mw = 9800 and Mw/Mn = 2.2.

### [Synthesis Example 16: Synthesis of Fluorine-Containing Polymer 9]

A fluorine-containing polymer was synthesized in the same manner as in Synthesis Example 10, except that MA-4FHB-OH was changed to MA-BTHB-OH and 4FIP-M was changed to HFIP-M. Thus, 10.0 g of a fluorine-containing polymer 9 having the following repeating units were obtained in a yield of 78%.

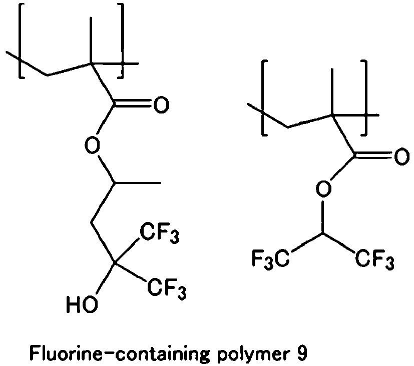

The GPC measurement results were as follows: Mw = 10000 and Mw/Mn = 1.9.

From the NMR measurement results, it was found that the compositional ratio of the structural units in the fluorine-containing polymer 9, expressed in mol%, was the structural unit derived from MA-BTHB-OH:the structural unit derived from HFIP-M = 53:47.

### [Synthesis Example 17: Synthesis of Fluorine-Containing Polymer 10]

A fluorine-containing polymer was synthesized in the same manner as in Synthesis Example 11, except that MA-4FHB-OH was changed to MA-BTHB-OH. Thus, 7.0 g of a fluorine-containing polymer 10 having the following repeating units was obtained in a yield of 75%.

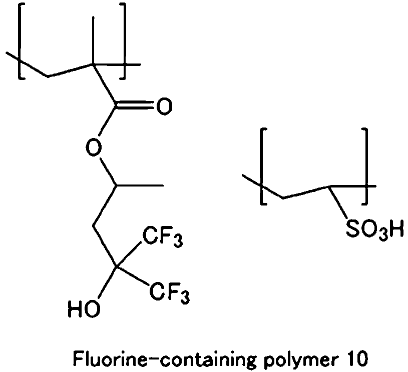

The GPC measurement results were as follows: Mw = 11000 and Mw/Mn = 1.9.

From the NMR measurement results, it was found that the compositional ratio of the structural units in the fluorine-containing polymer 10, expressed in mol%, was the structural unit derived from MA-BTHB-OH:the structural unit derived from vinylsulfonic acid = 58:42.

### [Synthesis Example 18: Synthesis of Fluorine-Containing Polymer 11]

A fluorine-containing polymer was synthesized in the same manner as in Synthesis Example 12, except that MA-4FHB-OH was changed to MA-BTHB-OH. Thus, 9.5 g of a fluorine-containing polymer 11 having the following repeating units was obtained in a yield of 80%.

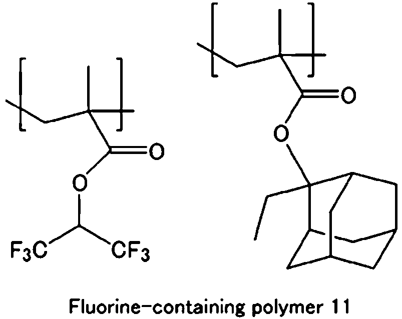

The GPC measurement results were as follows: Mw = 9000 and Mw/Mn = 2.2.

From the NMR measurement results, it was found that the compositional ratio of the structural units in the fluorine-containing polymer 11, expressed in mol%, was the structural unit derived from MA-BTHB-OH:the structural unit derived from EMOA = 54:46.

### [Synthesis Example 19: Fluorine-Containing Polymer 12]

A fluorine-containing polymer was synthesized in the same manner as in Synthesis Example 13, except that MA-4FHB-OH was changed to MA-BTHB-OH and 4FIP-M was changed to HFIP-M. Thus, 17.1 g of a fluorine-containing polymer 12 having the following repeating units was obtained in a yield of 85%.

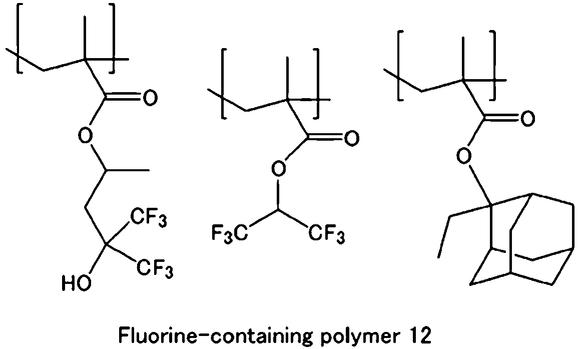

The GPC measurement results were as follows: Mw = 11000 and Mw/Mn = 2.3.

From the NMR measurement results, it was found that the compositional ratio of the structural units in the fluorine-containing polymer 12, expressed in mol%, was the structural unit derived from MA-BTHB-OH:the structural unit derived from HFIP-M:the structural unit derived from EMOA = 36:33:31.

### [Synthesis Example 20: Synthesis of Fluorine-Containing Polymer 13]

A fluorine-containing polymer was synthesized in the same manner as in Synthesis Example 8, except that MA-4FHB-OH was changed to MA-4FHB-NB. Thus, 5.1 g of a fluorine-containing polymer 13 having the following repeating unit was obtained in a yield of 70%.

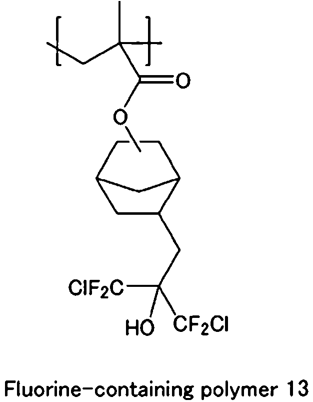

The GPC measurement results were as follows: Mw = 27200 and Mw/Mn = 2.0.

### [Synthesis Example 21: Synthesis of Fluorine-Containing Polymer 14]

A fluorine-containing polymer was synthesized in the same manner as in Synthesis Example 8, except that MA-4FHB-OH was changed to MA-4FHP-OH. Thus, 5.6 g of a fluorine-containing polymer 14 having the following repeating unit was obtained in a yield of 75%.

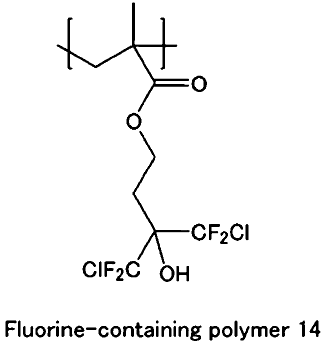

The GPC measurement results were as follows: Mw = 11600 and Mw/Mn = 2.1.

### [Synthesis Example 22: Synthesis of Fluorine-Containing Polymer 15]

A fluorine-containing polymer was synthesized in the same manner as in Synthesis Example 10, except that MA-4FHB-OH was changed to 4F-Iee. Thus, 5.1 g of a fluorine-containing polymer 15 having the following repeating units was obtained in a yield of 40%.

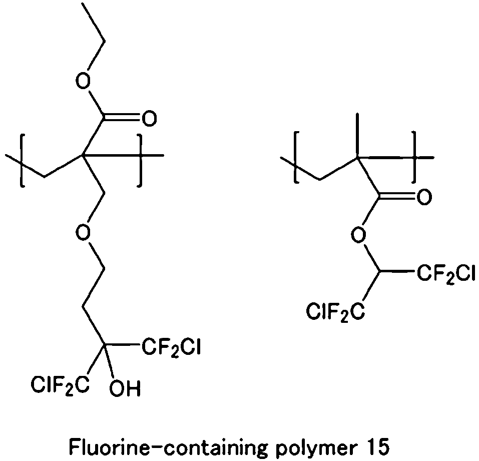

The GPC measurement results were as follows: Mw = 10800 and Mw/Mn = 1.8.

From the NMR measurement results, it was found that the compositional ratio of the structural units in the fluorine-containing polymer 3, expressed in mol%, was the structural unit derived from 4F-Iee:the structural unit derived from 4FIP-M = 52:48.

### [Synthesis Example 23: Synthesis of Fluorine-Containing Polymer 16]

A fluorine-containing polymer was synthesized in the same manner as in Synthesis Example 8, except that MA-4FHB-OH was changed to MA-MIB-4FA. Thus, 5.5 g of a fluorine-containing polymer 16 having the following repeating unit was obtained in a yield of 72%.

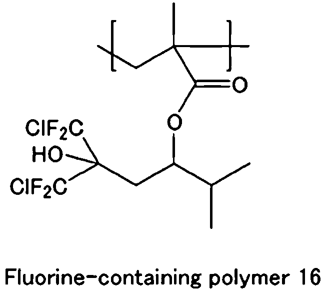

The GPC measurement results were as follows: Mw = 10000 and Mw/Mn = 2.0.

### [Synthesis Example 24: Synthesis of Fluorine-Containing Polymer 17]

A fluorine-containing polymer was synthesized in the same manner as in Synthesis Example 8, except that MA-4FHB-OH was changed to bis-4FIP form. Thus, 4.6 g of a fluorine-containing polymer 17 having the following repeating unit was obtained in a yield of 65%.

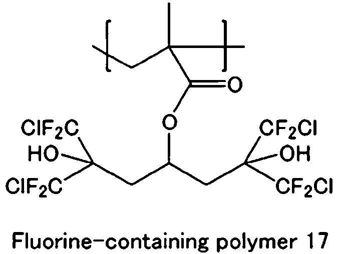

The GPC measurement results were as follows: Mw = 18200 and Mw/Mn = 2.6.

Table 1 shows the yield, Mw, and molecular weight distribution (Mw/Mn) of the fluorine-containing polymers 1 to 17.

**[Table 1]**

| Fluorine-containing polymer | Yield (%) | Mw | Mw/Mn |
|---|---|---|---|
| 1 | 80 | 13000 | 1.9 |
| 2 | 82 | 9000 | 2.1 |
| 3 | 84 | 9800 | 1.9 |
| 4 | 81 | 10800 | 1.8 |
| 5 | 85 | 9900 | 2.1 |
| 6 | 89 | 12000 | 2.3 |
| 7 | 80 | 12000 | 2.0 |
| 8 | 80 | 9000 | 2.1 |
| 9 | 78 | 10000 | 1.9 |
| 10 | 81 | 11000 | 1.9 |
| 11 | 80 | 9000 | 2.2 |
| 12 | 85 | 11000 | 2.3 |
| 13 | 70 | 27200 | 2.0 |
| 14 | 75 | 11600 | 2.1 |
| 15 | 40 | 10800 | 1.8 |
| 16 | 72 | 10000 | 2.0 |
| 17 | 65 | 18200 | 2.6 |

### <Decomposability Evaluation 1>

The decomposability of the fluorine-containing polymers 1 to 17 was determined using NMR by the following method.

To 1 g of each fluorine-containing polymer was added a 2.38 wt% aqueous TMAH solution (20 g), and ¹⁹F-NMR was measured. After stirring at room temperature for 24 hours or at 90°C for 24 hours, ¹⁹F-NMR was measured again, and if at least 50% of the ¹⁹F-NMR peak area before the stirring was replaced by new peaks with different chemical shift values, the chlorodifluoro group was determined to be decomposed.

The results are shown in Table 2.

**[Table 2]**

| Example | Fluorine-containing polymer | Decomposability | |
|---|---|---|---|
| | | Room temperature | 90°C |
| Example 1 | 1 | Not decomposed | Decomposed |
| Example 2 | 2 | Not decomposed | Decomposed |
| Example 3 | 3 | Not decomposed | Decomposed |
| Example 4 | 4 | Not decomposed | Decomposed |
| Example 5 | 5 | Not decomposed | Decomposed |
| Example 6 | 6 | Not decomposed | Decomposed |
| Example 7 | 13 | Not decomposed | Decomposed |
| Example 8 | 14 | Not decomposed | Decomposed |
| Example 9 | 15 | Not decomposed | Decomposed |
| Example 10 | 16 | Not decomposed | Decomposed |
| Example 11 | 17 | Not decomposed | Decomposed |
| Comparative Example 1 | 7 | Not decomposed | Not decomposed |
| Comparative Example 2 | 8 | Not decomposed | Not decomposed |
| Comparative Example 3 | 9 | Not decomposed | Not decomposed |
| Comparative Example 4 | 10 | Not decomposed | Not decomposed |
| Comparative Example 5 | 11 | Not decomposed | Not decomposed |
| Comparative Example 6 | 12 | Not decomposed | Not decomposed |

It was demonstrated that the fluorine-containing polymers (Comparative Examples 1 to 6) containing MA-BTHB-OH or HFIP-M as a repeating unit, each having a trifluoromethyl group, were not decomposed under either room temperature or heating conditions, whereas the fluorine-containing polymers (Examples 1 to 11) containing MA-4FHB-OH, 4FIP-M, or the like as a repeating unit, each having a chlorodifluoro group, underwent decomposition of the chlorodifluoro group at 90°C.

These results provide fluorine-containing polymers that can be decomposed at a lower temperature. In a photolithography step, the developed fluorine-containing polymers can be decomposed at a lower temperature, and therefore they are expected to contribute to energy reduction in semiconductor processes.

### <Decomposability Evaluation 2>

The decomposability of the fluorine-containing polymers 1 to 17 was evaluated by subjecting the fluorine-containing polymers to a treatment under the following conditions and measuring the concentration of generated fluoride ions and chloride ions.

To 1 g of each fluorine-containing polymer was added a 2.38 wt% aqueous TMAH solution (20 g), and the mixture was stirred at 90°C for 24 hours to obtain a solution. To 21 g of each solution was added 20 g of diisopropyl ether (IPE), and after stirring for 5 minutes, the mixture was allowed to stand and subjected to liquid-liquid separation to remove the organic matter. Then, the fluoride and chloride ions contained in the aqueous layer were measured by ion chromatography. The ion chromatographic apparatus used was "ICS-2100" manufactured by Thermo Fisher Scientific Inc., the column used was IonPac CS16 RFIC (5 × 250 mm) manufactured by Thermo Fisher Scientific Inc., the eluent used was an aqueous KOH solution, and the detector used was an electrical conductivity detector (under these conditions, fluoride ions and chloride ions were detected at 8.8 min and 13.3 min, respectively).

From the measurement results, the concentration of fluoride ions and chloride ions was determined using calibration curves. The results are shown in Table 3.

**[Table 3]**

| Example | Fluorine-containing polymer | Fluoride ion concentration (wtppm) | Chloride ion concentration (wtppm) |
|---|---|---|---|
| Example 1 | 1 | 11000 | 10300 |
| Example 2 | 2 | 13500 | 12600 |
| Example 3 | 3 | 12200 | 11300 |
| Example 4 | 4 | 8300 | 7700 |
| Example 5 | 5 | 7000 | 6500 |
| Example 6 | 6 | 8500 | 8000 |
| Example 7 | 13 | 9000 | 8500 |
| Example 8 | 14 | 11000 | 10000 |
| Example 9 | 15 | 11000 | 10500 |
| Example 10 | 16 | 9800 | 9000 |
| Example 11 | 17 | 13000 | 12000 |
| Comparative Example 1 | 7 | <10 | <10 |
| Comparative Example 2 | 8 | <10 | <10 |
| Comparative Example 3 | 9 | <10 | <10 |
| Comparative Example 4 | 10 | <10 | <10 |
| Comparative Example 5 | 11 | <10 | <10 |
| Comparative Example 6 | 12 | <10 | <10 |

As shown in Table 3, it was confirmed that the fluorine-containing polymers (Examples 1 to 11) containing MA-4FHB-OH or 4FIP-M as a repeating unit, each having a chlorodifluoro group, underwent decomposition of the chlorodifluoro group into fluoride ions and chloride ions under basic conditions at 90°C.

From these results, the fluorine-containing polymers according to the examples of the present disclosure were found to be fluorine-containing polymers that can be decomposed at a lower temperature than conventional fluorine-containing polymers.

Next, in order to determine whether the fluorine-containing polymers of the present disclosure can be used for resist films, upper layer films, and the like in photolithography, the following evaluations were conducted: <Water Repellency Evaluation>, <Water Insolubility Evaluation>, <Alkaline Developer Solubility Evaluation>, and <193 nm Transmittance Evaluation>.

### <Water Repellency Evaluation>

An amount of 1 g of each fluorine-containing polymer was added to PGMEA (3 g) to prepare a composition. Thereafter, the composition was applied onto a 4-inch silicon substrate by a spinner and then dried on a hot plate heated to 80°C for 3 minutes to form a film having a film thickness of 2 to 3 µm.

### [Measurement of Static Contact Angle]

A water droplet was placed on the film on the silicon substrate, and the static contact angle was measured by the droplet method using a contact angle meter (manufactured by Kyowa Interface Science Co., Ltd.). In the present invention, the static contact angle refers to the angle formed with the film surface when a water droplet is placed on the film surface. The results are shown in Table 4.

### [Measurement of Hysteresis (Dynamic Advancing Angle - Dynamic Receding Angle)]

A water droplet was placed on the film on the silicon substrate, and the hysteresis (dynamic advancing angle - dynamic receding angle) was measured by the extension/contraction method using a contact angle meter (manufactured by Kyowa Interface Science Co., Ltd.). In the present invention, the dynamic receding angle refers to the contact angle when the water droplet is aspirated using a needle or the like so that the droplet contracts, the dynamic advancing angle refers to the contact angle when the water droplet is dispensed using a needle or the like so that the droplet expands, and the hysteresis refers to the difference between the dynamic contact angle and the dynamic receding angle. The results are shown in Table 4.

### [Measurement of Sliding Angle]

A water droplet (50 µL) was placed on the film on the silicon substrate, and the sliding angle was measured using a contact angle meter (manufactured by Kyowa Interface Science Co., Ltd.). The sliding angle refers to the angle of inclination of the silicon substrate at which the water droplet starts to move. The results are shown in Table 4.

**[Table 4]**

| Example | Fluorine-containing polymer | Static contact angle (°) | Sliding angle (°) | Hysteresis (°) |
|---|---|---|---|---|
| Example 1 | 1 | 88 | 17 | 18 |
| Example 2 | 2 | 101 | 6 | 7 |
| Example 3 | 3 | 95 | 11 | 11 |
| Example 4 | 4 | 85 | 20 | 21 |
| Example 5 | 5 | 88 | 18 | 19 |
| Example 6 | 6 | 90 | 17 | 18 |
| Example 7 | 13 | 81 | 20 | 20 |
| Example 8 | 14 | 82 | 15 | 16 |
| Example 9 | 15 | 98 | 9 | 9 |
| Example 10 | 16 | 91 | 15 | 16 |
| Example 11 | 17 | 88 | 17 | 18 |
| Comparative Example 1 | 7 | 88 | 15 | 15 |
| Comparative Example 2 | 8 | 106 | 9 | 11 |
| Comparative Example 3 | 9 | 97 | 10 | 10 |
| Comparative Example 4 | 10 | 84 | 21 | 22 |
| Comparative Example 5 | 11 | 88 | 18 | 20 |
| Comparative Example 6 | 12 | 89 | 18 | 19 |

Examples 1 to 11 exhibited static contact angles, sliding angles, and hystereses comparable to those of Comparative Examples 1 to 6, confirming that the fluorine-containing polymers containing MA-4FHB-OH or 4FIP-M as a repeating unit, each having a chlorodifluoro group, also possess comparable water repellency.

### <Water Insolubility Evaluation>

The fluorine-containing polymer 1 (1 g) was added to PGMEA (3 g) to prepare a composition. Thereafter, the composition was applied onto a 4-inch silicon substrate by a spinner and then dried on a hot plate heated to 80°C for 3 minutes to form a film having a film thickness of 3 µm. Thereafter, the film was immersed in ion-exchanged water for 1 minute, after which the film thickness on the silicon substrate was measured. The film thickness was found to be 3 µm, confirming that the film is insoluble in ion-exchanged water.

### <Alkaline Developer Solubility Evaluation>

The fluorine-containing polymer 1 (1 g) was added to PGMEA (3 g) to prepare a composition. Thereafter, the composition was applied onto a 4-inch silicon substrate by a spinner and then dried on a hot plate heated to 80°C for 3 minutes to form a film having a film thickness of 3 µm. Thereafter, the film was immersed in a 2.38 wt% aqueous solution of tetramethylammonium hydroxide (TMAH) for 1 minute. The film on the silicon substrate was found to be removed, confirming that the film was dissolved in the TMAH solution.

### <193 nm Transmittance Evaluation>

The fluorine-containing polymer 1 (1 g) was added to PGMEA (19 g) to prepare a composition. Thereafter, the composition was applied onto 4-inch quartz glass by a spinner and then dried on a hot plate heated to 80°C for 3 minutes to form a film having a film thickness of 225 nm. Thereafter, the film was irradiated with light at 193 nm using the transmittance measurement mode of ArFES-3500LDLS (manufactured by Litho Tech Japan Corporation) to measure the transmittance. The transmittance at 193 nm was 97%, confirming that the film sufficiently transmits light at 193 nm. The film thickness after the irradiation was measured and found to be 225 nm, remaining unchanged before and after the irradiation, which confirms that the film is inert to light at 193 nm.

From the above results, it was found that the fluorine-containing polymers of the present disclosure can be suitably used for resist films, upper layer films, and the like in photolithography.

In summary of the above results, it was found that the present disclosure provides a fluorine-containing polymer that can be decomposed at a lower temperature than conventional fluorine-containing polymers. Furthermore, it was found that the fluorine-containing polymer of the present disclosure can be suitably used for resist films, upper layer films, and the like in photolithography, and the waste material derived from the fluorine-containing polymer of the present disclosure can be disposed of by precipitation, adsorption, activated sludge, or other methods that are simpler than combustion, thereby contributing to energy reduction in semiconductor processes.

## Claims

1. A fluorine-containing polymer, comprising a repeating unit represented by the following formula (1) or (1)': 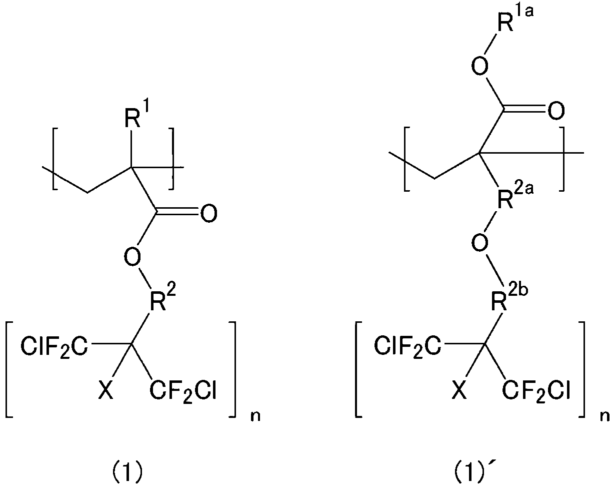
wherein, in formula (1), R¹ is a hydrogen atom, a fluorine atom, a chlorine atom, or a C1-C10 linear or C3-C10 branched alkyl group, some or all of hydrogen atoms bonded to carbon atoms in the alkyl group being optionally replaced by fluorine atoms;
in formula (1), R² is a single bond, an alkylene group optionally having a linear, branched, or cyclic structure, an aromatic ring, an ester, a carbonyl, an ether, an amide, an amine, or a composite substituent thereof, a part of which is optionally fluorinated, chlorinated, or both fluorinated and chlorinated;
in formula (1)', R^{1a} is a C1-C10 linear or C3-C10 branched alkyl group, some or all of hydrogen atoms bonded to carbon atoms in the alkyl group being optionally replaced by fluorine atoms, chlorine atoms, or both;
in formula (1)', R^{2a} is an alkylene group optionally having a linear, branched, or cyclic structure, an aromatic ring, an ester, a carbonyl, an ether, an amide, an amine, or a composite substituent thereof, a part of which is optionally fluorinated, chlorinated, or both fluorinated and chlorinated;
in formula (1)', R^{2b} is an alkylene group optionally having a linear, branched, or cyclic structure, an aromatic ring, an ester, a carbonyl, an ether, an amide, an amine, or a composite substituent thereof, a part of which is optionally fluorinated, chlorinated, or both fluorinated and chlorinated;
in formulae (1) and (1)', X is a hydroxy group, an alkoxy group, an oxycarbonyl group, an oxysulfonyl group, an oxycarboxy group, or a hydrogen atom; and
in formulae (1) and (1)', n is a natural number of 1 to 3.

2. The fluorine-containing polymer according to claim 1,
wherein the repeating units represented by formulae (1) and (1)' comprise repeating units represented by the following formulae (2) and (2)', respectively.

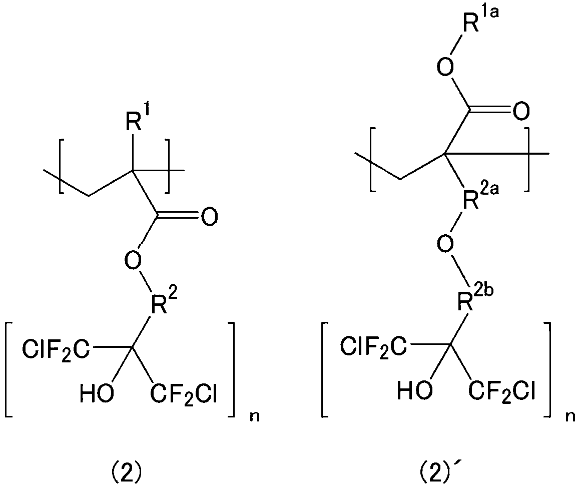

3. The fluorine-containing polymer according to claim 2, comprising a repeating unit represented by the following formula (3): 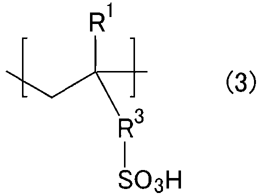 wherein, in formula (3), R³ is a single bond, an alkylene group optionally having a linear, branched, or cyclic structure, or an aromatic ring, a part of which optionally contains an ester bond, a carbonyl bond, an ether bond, an amide bond, an acetal bond, a hydroxy group, an amino group, a fluorine atom, or a chlorine atom, and is optionally a composite substituent thereof.

4. The fluorine-containing polymer according to claim 1,
wherein the repeating units represented by formulae (1) and (1)' comprise repeating units represented by the following formulae (4) and (4)', respectively.

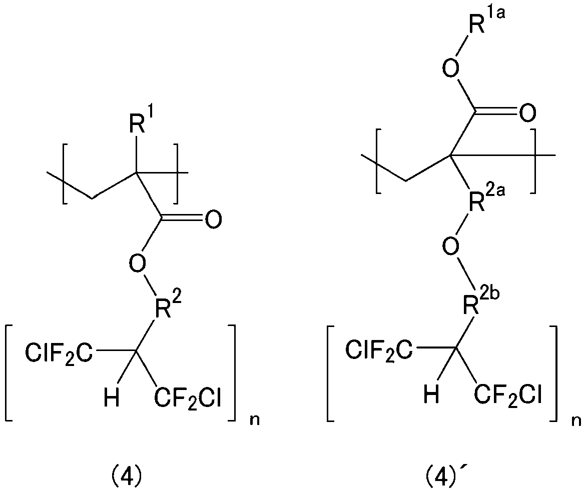

5. The fluorine-containing polymer according to claim 4, comprising a repeating unit represented by the following formula (5): 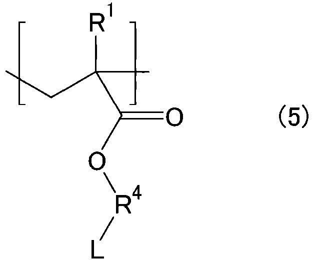
wherein, in formula (5), R⁴ is a single bond, an alkylene group optionally having a linear, branched, or cyclic structure, or an aromatic ring, a part of which optionally contains an ester bond, a carbonyl bond, an ether bond, an amide bond, an acetal bond, a hydroxy group, an amino group, a fluorine atom, or a chlorine atom, and is optionally a composite substituent thereof; and
L is an acid-dissociable group.

6. The fluorine-containing polymer according to claim 4,
wherein the repeating units represented by formulae (1) and (1)' comprise repeating units represented by the following formulae (2) and (2)', respectively.

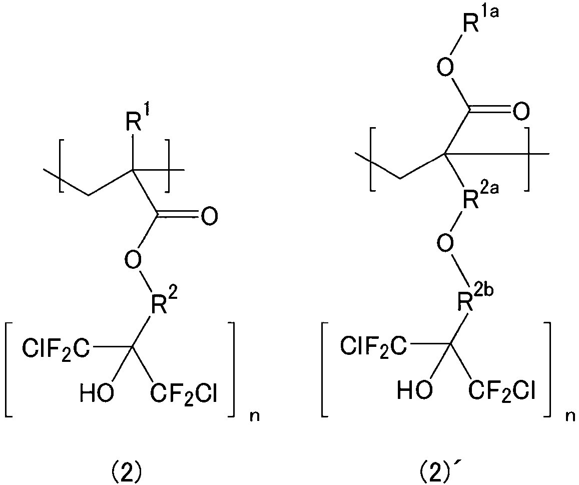

7. The fluorine-containing polymer according to claim 6, comprising a repeating unit represented by the following formula (5): 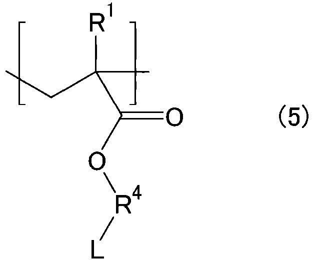
wherein, in formula (5), R⁴ is a single bond, an alkylene group optionally having a linear, branched, or cyclic structure, or an aromatic ring, a part of which optionally contains an ester bond, a carbonyl bond, an ether bond, an amide bond, an acetal bond, a hydroxy group, an amino group, a fluorine atom, or a chlorine atom, and is optionally a composite substituent thereof; and
L is an acid-dissociable group.

8. A composition for forming a fluorine-containing resin film, comprising the fluorine-containing polymer according to any one of claims 1 to 7.

9. A fluorine-containing resin film, comprising a coating film of the composition for forming a fluorine-containing resin film according to claim 8.

10. A composition for forming a resist pattern, comprising the fluorine-containing polymer according to any one of claims 1 to 7, an acid generator, and a solvent.

11. A method for forming a resist pattern, the method comprising:
a step of providing the composition for forming a resist pattern according to claim 10;
a film formation step including applying the composition for forming a resist pattern onto a substrate to form a film;
an exposure step including exposing the film to electromagnetic waves or high-energy rays having a wavelength of 300 nm or less through a photomask to transfer a pattern of the photomask to the film; and
a development step including developing the film using a developer to obtain the pattern.

12. A composition for forming a resist upper layer film, comprising the fluorine-containing polymer according to any one of claims 1 to 7 and a solvent.

13. A method for forming a resist pattern, the method comprising:
a step of providing the composition for forming a resist upper layer film according to claim 12;
a film formation step including applying the composition for forming a resist upper layer film onto a resist film to form a resist upper layer film;
an exposure step including exposing the resist upper layer film and the resist film to electromagnetic waves or high-energy rays having a wavelength of 300 nm or less through a photomask to transfer a pattern of the photomask to the resist film; and
a development step including removing the resist upper layer film and developing the resist film using a developer to obtain the pattern.

14. A method for decomposing the fluorine-containing polymer according to any one of claims 1 to 7, the method comprising heating the fluorine-containing polymer at a temperature in a range of 50°C to 200°C.

15. A fluorine-containing polymerizable monomer, represented by the following formula (6) or (6)': 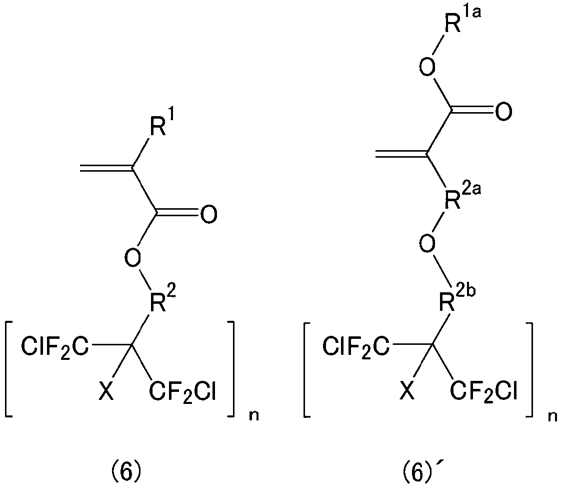
wherein, in formula (6), R¹ is a hydrogen atom, a fluorine atom, a chlorine atom, or a C1-C10 linear or C3-C10 branched alkyl group, some or all of hydrogen atoms bonded to carbon atoms in the alkyl group being optionally replaced by fluorine atoms;
in formula (6), R² is a single bond, an alkylene group optionally having a linear, branched, or cyclic structure, an aromatic ring, an ester, a carbonyl, an ether, an amide, an amine, or a composite substituent thereof, a part of which is optionally fluorinated, chlorinated, or both fluorinated and chlorinated;
in formula (6)', R^{1a} is a C1-C10 linear or C3-C10 branched alkyl group, some or all of hydrogen atoms bonded to carbon atoms in the alkyl group being optionally replaced by fluorine atoms, chlorine atoms, or both;
in formula (6)', R^{2a} is an alkylene group optionally having a linear, branched, or cyclic structure, an aromatic ring, an ester, a carbonyl, an ether, an amide, an amine, or a composite substituent thereof, a part of which is optionally fluorinated, chlorinated, or both fluorinated and chlorinated;
in formula (6)', R^{2b} is an alkylene group optionally having a linear, branched, or cyclic structure, an aromatic ring, an ester, a carbonyl, an ether, an amide, an amine, or a composite substituent thereof, a part of which is optionally fluorinated, chlorinated, or both fluorinated and chlorinated;
in formulae (6) and (6)', X is a hydroxy group, an alkoxy group, an oxycarbonyl group, an oxysulfonyl group, an oxycarboxy group, or a hydrogen atom; and
in formulae (6) and (6)', n is a natural number of 1 to 3.

16. A compound, represented by the following formula (7): 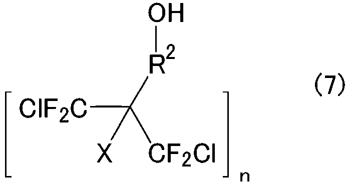
wherein, in formula (7), R² is a single bond, an alkylene group optionally having a linear, branched, or cyclic structure, an aromatic ring, an ester, a carbonyl, an ether, an amide, an amine, or a composite substituent thereof, a part of which is optionally fluorinated, chlorinated, or both fluorinated and chlorinated;
X is a hydroxy group, an alkoxy group, an oxycarbonyl group, an oxysulfonyl group, an oxycarboxy group, or a hydrogen atom; and
n is a natural number of 1 to 3.

17. A method for producing a fluorine-containing polymerizable monomer represented by the following formula (6), the method comprising:
a step of providing a compound represented by the following formula (7); and
a step of reacting the compound represented by formula (7) with at least one selected from the group consisting of an acid halide, an acid anhydride, an ester, and a carboxylic acid: 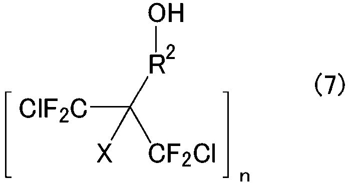
wherein, in formula (7), R² is a single bond, an alkylene group optionally having a linear, branched, or cyclic structure, an aromatic ring, an ester, a carbonyl, an ether, an amide, an amine, or a composite substituent thereof, a part of which is optionally fluorinated, chlorinated, or both fluorinated and chlorinated;
X is a hydroxy group, an alkoxy group, an oxycarbonyl group, an oxysulfonyl group, an oxycarboxy group, or a hydrogen atom; and
n is a natural number of 1 to 3, 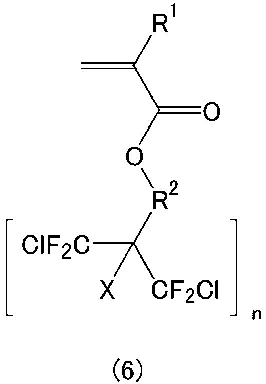
wherein, in formula (6), R¹ is a hydrogen atom, a fluorine atom, a chlorine atom, or a C1-C10 linear or C3-C10 branched alkyl group, some or all of hydrogen atoms bonded to carbon atoms in the alkyl group being optionally replaced by fluorine atoms;
R² is a single bond, an alkylene group optionally having a linear, branched, or cyclic structure, an aromatic ring, an ester, a carbonyl, an ether, an amide, an amine, or a composite substituent thereof, a part of which is optionally fluorinated, chlorinated, or both fluorinated and chlorinated;
X is a hydroxy group, an alkoxy group, an oxycarbonyl group, an oxysulfonyl group, an oxycarboxy group, or a hydrogen atom; and
n is a natural number of 1 to 3.

18. A method for producing a fluorine-containing polymerizable monomer represented by the following formula (6)', the method comprising:
a step of providing a compound represented by the following formula (8); and
a step of reacting the compound represented by formula (8) with a compound represented by the following formula (9): 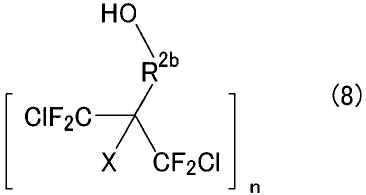
wherein, in formula (8), R^{2b} is an alkylene group optionally having a linear, branched, or cyclic structure, an aromatic ring, an ester, a carbonyl, an ether, an amide, an amine, or a composite substituent thereof, a part of which is optionally fluorinated, chlorinated, or both fluorinated and chlorinated;
in formula (8), X is a hydroxy group, an alkoxy group, an oxycarbonyl group, an oxysulfonyl group, an oxycarboxy group, or a hydrogen atom; and
in formula (8), n is a natural number of 1 to 3, 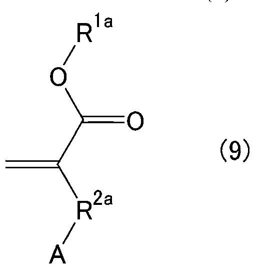
wherein, in formula (9), R^{1a} is a C1-C10 linear or C3-C10 branched alkyl group, some or all of hydrogen atoms bonded to carbon atoms in the alkyl group being optionally replaced by fluorine atoms, chlorine atoms, or both;
in formula (9), R^{2a} is an alkylene group optionally having a linear, branched, or cyclic structure, an aromatic ring, an ester, a carbonyl, an ether, an amide, an amine, or a composite substituent thereof, a part of which is optionally fluorinated, chlorinated, or both fluorinated and chlorinated; and
in formula (9), A is a leaving group, 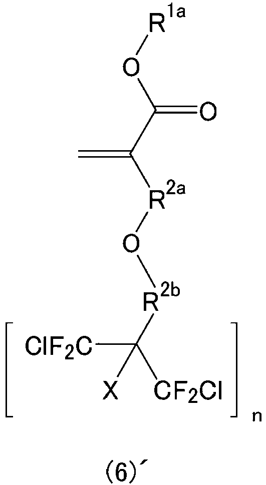
wherein, in formula (6)', R^{1a} is a C1-C10 linear or C3-C10 branched alkyl group, some or all of hydrogen atoms bonded to carbon atoms in the alkyl group being optionally replaced by fluorine atoms, chlorine atoms, or both;
in formula (6)', R^{2a} is an alkylene group optionally having a linear, branched, or cyclic structure, an aromatic ring, an ester, a carbonyl, an ether, an amide, an amine, or a composite substituent thereof, a part of which is optionally fluorinated, chlorinated, or both fluorinated and chlorinated;
in formula (6)', R^{2b} is an alkylene group optionally having a linear, branched, or cyclic structure, an aromatic ring, an ester, a carbonyl, an ether, an amide, an amine, or a composite substituent thereof, a part of which is optionally fluorinated, chlorinated, or both fluorinated and chlorinated;
in formula (6)', X is a hydroxy group, an alkoxy group, an oxycarbonyl group, an oxysulfonyl group, an oxycarboxy group, or a hydrogen atom; and
in formula (6)', n is a natural number of 1 to 3.

19. A method for producing a fluorine-containing polymerizable monomer represented by the following formula (6)', the method comprising:
a step of providing a compound represented by the following formula (10); and
a step of reacting the compound represented by formula (10) with a compound represented by the following formula (11): 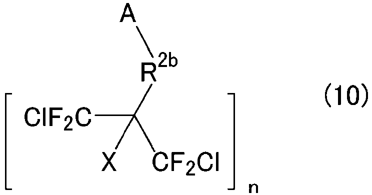
wherein, in formula (10), R^{2b} is an alkylene group optionally having a linear, branched, or cyclic structure, an aromatic ring, an ester, a carbonyl, an ether, an amide, an amine, or a composite substituent thereof, a part of which is optionally fluorinated, chlorinated, or both fluorinated and chlorinated;
in formula (10), X is a hydroxy group, an alkoxy group, an oxycarbonyl group, an oxysulfonyl group, an oxycarboxy group, or a hydrogen atom;
in formula (10), A is a leaving group; and
in formula (10), n is a natural number of 1 to 3, 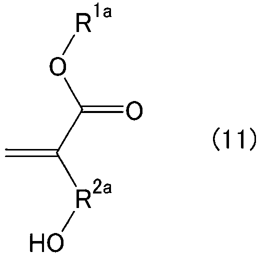
wherein, in formula (11), R^{1a} is a C1-C10 linear or C3-C10 branched alkyl group, some or all of hydrogen atoms bonded to carbon atoms in the alkyl group being optionally replaced by fluorine atoms, chlorine atoms, or both; and
in formula (11), R^{2a} is an alkylene group optionally having a linear, branched, or cyclic structure, an aromatic ring, an ester, a carbonyl, an ether, an amide, an amine, or a composite substituent thereof, a part of which is optionally fluorinated, chlorinated, or both fluorinated and chlorinated, 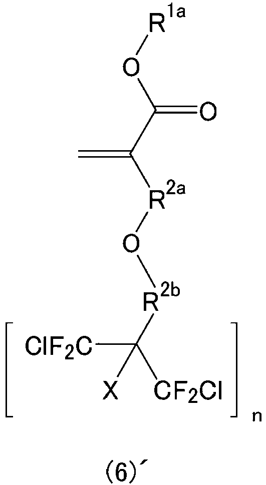
wherein, in formula (6)', R^{1a} is a C1-C10 linear or C3-C10 branched alkyl group, some or all of hydrogen atoms bonded to carbon atoms in the alkyl group being optionally replaced by fluorine atoms, chlorine atoms, or both;
in formula (6)', R^{2a} is an alkylene group optionally having a linear, branched, or cyclic structure, an aromatic ring, an ester, a carbonyl, an ether, an amide, an amine, or a composite substituent thereof, a part of which is optionally fluorinated, chlorinated, or both fluorinated and chlorinated;
in formula (6)', R^{2b} is an alkylene group optionally having a linear, branched, or cyclic structure, an aromatic ring, an ester, a carbonyl, an ether, an amide, an amine, or a composite substituent thereof, a part of which is optionally fluorinated, chlorinated, or both fluorinated and chlorinated;
in formula (6)', X is a hydroxy group, an alkoxy group, an oxycarbonyl group, an oxysulfonyl group, an oxycarboxy group, or a hydrogen atom; and
in formula (6)', n is a natural number of 1 to 3.
